(19) Europäisches Patentamt / European Patent Office / Office européen des brevets

(11) EP 3 973 991 A1

(12) EUROPEAN PATENT APPLICATION
published in accordance with Art. 153(4) EPC

(43) Date of publication:
30.03.2022 Bulletin 2022/13

(21) Application number: 20806566.4

(22) Date of filing: 07.05.2020

(51) International Patent Classification (IPC):
*A61K 45/00* (2006.01) *A61K 45/06* (2006.01)
*A61P 35/00* (2006.01) *A61P 43/00* (2006.01)
*C07K 16/18* (2006.01) *C12N 15/113* (2010.01)
*C12Q 1/02* (2006.01) *C12N 9/99* (2006.01)
*G01N 33/15* (2006.01) *G01N 33/50* (2006.01)
*G01N 33/53* (2006.01) *A61K 31/12* (2006.01)
*A61K 31/135* (2006.01) *A61K 31/137* (2006.01)
*A61K 31/198* (2006.01) *A61K 31/4453* (2006.01)
*A61K 31/635* (2006.01)

(52) Cooperative Patent Classification (CPC):
**A61K 31/12; A61K 31/135; A61K 31/137; A61K 31/198; A61K 31/4453; A61K 31/635; A61K 45/00; A61K 45/06; A61P 35/00; A61P 43/00; C07K 16/18; C12N 9/99; C12N 15/113; C12Q 1/02; G01N 33/15;** (Cont.)

(86) International application number:
PCT/JP2020/018572

(87) International publication number:
WO 2020/230701 (19.11.2020 Gazette 2020/47)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 14.05.2019 JP 2019091358
01.11.2019 JP 2019200094

(71) Applicant: Keio University
Tokyo, 108-8345 (JP)

(72) Inventors:
- SAYA, Hideyuki
Tokyo 160-8582 (JP)
- NAGANO, Osamu
Tokyo 160-8582 (JP)
- OTSUKI, Yuji
Tokyo 160-8582 (JP)

(74) Representative: Gill Jennings & Every LLP
The Broadgate Tower
20 Primrose Street
London EC2A 2ES (GB)

# (54) ANTI-TUMOR AGENT AND COMPOUNDING AGENT

(57) [Problem] To provide novel anti-tumor agents and combination drugs.

[Means to solve] To provide an anti-tumor agent containing, as an active ingredient, a glutathione level reducer or a glutathione S-transferase inhibitor, the anti-tumor agent being adapted to be administered simultaneously with an effective amount of a compound (II); an anti-tumor agent including a compound (II) as an active ingredient, the anti-tumor agent being adapted to be administered simultaneously with an effective amount of a glutathione level reducer or a glutathione S-transferase inhibitor; or an anti-tumor agent containing, as active ingredients, a compound (II) and a glutathione level reducer or a glutathione S-transferase inhibitor, where $R^5$ is a linear or branched C1-6 alkyl group, $R^6$ is hydrogen or halogen, $R^7$ is a linear or branched C1-6 alkyl group optionally substituted with a substituent, the substituent being hydroxy or phenyl, and $R^8$ is hydrogen or halogen.

EP 3 973 991 A1

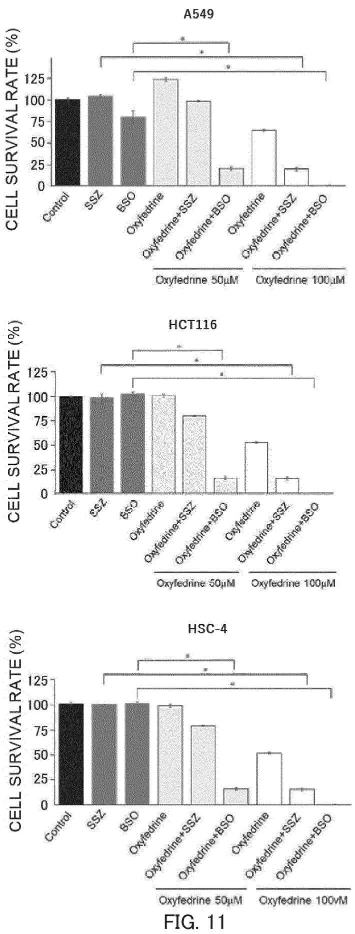


FIG. 11

(52) Cooperative Patent Classification (CPC): (Cont.)
**G01N 33/50; G01N 33/53**

## Description

### Technical Field

The present invention relates to anti-tumor agents and combination drugs.

### Background art

In cancer treatments, the presence of cells resistant to chemo- and/or radiotherapy is attributed to relapse as well as to metastasis and interferes with the treatment of cancers. Cancer stem cells have drawn attention as treatment-resistant cells in recent years. Since cancer stem cells are highly resistant to various stresses, the development of drugs that target cancer stem cells is a matter of urgency for complete cure of cancers; however, investigations on the molecular mechanisms of stress resistance of cancer stem cells for the development of therapeutic targeting of cancer stem cells have only just started.

CD44, one of the markers for epithelial cancer stem cells, is known as a molecule involved in stress resistance of cancer stem cells (Cancer Cell. 2011 Mar. 8; 19(3): 387-400). CD44 has a splice variant form (hereinafter, CD44v), which stabilizes the expression of the cystine transporter xCT on cell membranes. xCT has a function of uptaking cystine into cells and the uptaken cystine is used for the production of glutathione (GSH); thus, the GSH content increases in cells with a high CD44v expression. Since GSH has a strong anti-oxidative effect and plays a role in reducing stresses of cells, it has been thought that cancer stem cells with a high CD44v expression are resistant to cancer treatments.

Sulfasalazine (also known as salazosulfapyridine, salazopyrin, and salicylazosulfapyridine) is used in treating ulcerative colitis and rheumatoid arthritis. It is an acidic azo compound of sulfapyridine and 5-aminosalicylic acid (5-ASA). When administered orally, sulfasalazine is metabolized into sulfapyridine and 5-aminosalicylic acid (5-ASA) by intestinal bacteria in the intestine. Particularly, for the aforementioned diseases, 5-ASA is understood as the primary active ingredient.

In recent years, it has been revealed that intact sulfasalazine before metabolic degradation exerts an inhibitory effect on xCT and is an effective anti-tumor agent (Leukemia vol. 15, pp. 1633-1640, 2001). This means that when sulfasalazine is added to cancer cells, uptake of cystine into cells by xCT is suppressed and the glutathione production is reduced. Consequently, the oxidative stress resistance of the cancer cells is reduced, and their sensitivity to anti-tumor agents is increased.

Sulfasalazine, which exerts an inhibitory effect on xCT, is known to effectively suppress the growth of cancer stem cells with a high CD44v expression as well (JP-A-2012-144498).

### Summary of the Invention

An object of the present invention is to provide novel anti-tumor agents and combination drugs.

The inventors have found that sulfasalazine alone exerts an anti-tumor effect on tumors that are mostly composed of undifferentiated tumor cells; however, it does not exert the effect of the reduction of the overall tumor volume of differentiation-type tumors that contain tumor cells exhibiting differentiated traits although it decreases the number of cancer stem cells that express CD44v at a high level in the tumors. Accordingly, the inventors made intensive efforts which had been directed toward the development of anti-tumor agents for the tumor cells in the differentiation-type tumors on which sulfasalazine does not exert anti-tumor effects, to obtain anti-tumor agents for such differentiation-type tumors. As a result, the inventors found that by combined use of an aldehyde dehydrogenase inhibitor or oxyfedrine and sulfasalazine or L-buthionine-sulfoximine, a remarkable anti-tumor effect was exerted on tumor cells compared to sulfasalazine or L-buthionine-sulfoximine alone, leading to the completion of the present invention.

An aspect of the present invention is an anti-tumor agent including, as an active ingredient, a glutathione level reducer or a glutathione S-transferase inhibitor, the anti-tumor agent being administered simultaneously with an effective amount of an aldehyde dehydrogenase inhibitor, or a compound (II) below or a pharmacologically acceptable salt thereof:

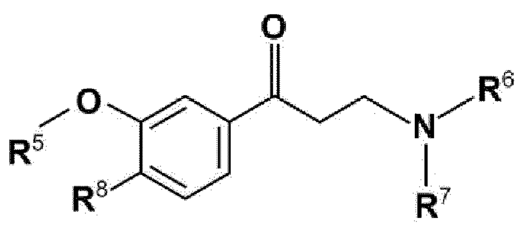

(II)

wherein $R^5$ is a linear or branched C1-6 alkyl group, $R^6$ is hydrogen or halogen, $R^7$ is a linear or branched C1-6 alkyl group optionally substituted with a substituent, the substituent being hydroxy or phenyl, and $R^8$ is hydrogen or halogen.

Another aspect of the present invention is an anti-tumor agent including, as an active ingredient, an aldehyde dehydrogenase inhibitor, or a compound (II) below or a pharmacologically acceptable salt thereof, the anti-tumor agent being administered simultaneously with an effective amount of a glutathione level reducer or a glutathione S-transferase inhibitor:

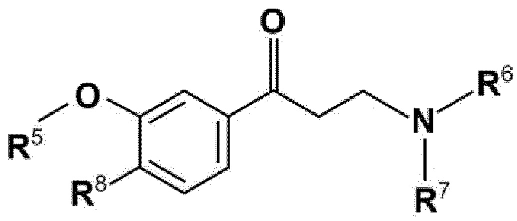

(I I)

wherein $R^5$ is a linear or branched C1-6 alkyl group, $R^2$ and $R^3$ are each independently selected from linear and branched C1-6 alkyl groups, or $R^2$ and $R^3$ form a 4-, 5-, 6-, or 7-membered azacycloalkyl group together with the neighboring nitrogen as a heteroatom, and $R^4$ is hydrogen or halogen.

In any of the aforementioned anti-tumor agents, the glutathione level reducer may inhibit activity of any one of xCT, thioredoxin-1 (TRX-1), glutamate-cysteine ligase (GCL) (EC 6.3.2.2) (also called γ-glutamylcysteine synthetase), and glutathione synthetase (EC 6.3.2.3). The glutathione level reducer may be an xCT inhibitor or a GCL inhibitor, or may be sulfasalazine or L-buthionine-sulfoximine. The compound represented by the formula (II) may be oxyfedrine.

A further aspect of the present invention is a combination drug including, as active ingredients, an aldehyde dehydrogenase inhibitor, or a compound (II) below or a pharmacologically acceptable salt thereof; and a glutathione level reducer or a glutathione S-transferase inhibitor:

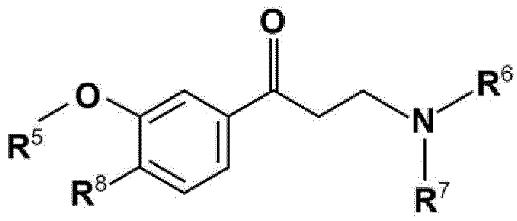

(I I)

wherein $R^5$ is a linear or branched C1-6 alkyl group, $R^6$ is hydrogen or halogen, $R^7$ is a linear or branched C1-6 alkyl group optionally substituted with a substituent, the substituent being hydroxy or phenyl, and $R^8$ is hydrogen or halogen.

In the aforementioned combination drugs, the glutathione level reducer may inhibit activity of any one of xCT, thioredoxin-1 (TRX-1), glutamate-cysteine ligase (GCL) (EC 6.3.2.2) (also called γ-glutamylcysteine synthetase), and glutathione synthetase (EC 6.3.2.3). The glutathione level reducer may be an xCT inhibitor or a GCL inhibitor, or may be sulfasalazine or a derivative thereof, or L-buthionine-sulfoximine. The compound represented by the formula (II) may be oxyfedrine.

A further aspect of the present invention is an anti-tumor agent including any one of the aforementioned combination drugs.

Any one of the aforementioned anti-tumor agents may be against a tumor containing a tumor cell resistant to a glutathione level reducer or a glutathione S-transferase inhibitor. The tumor cell may have a high expression of aldehyde dehydrogenase. The tumor may further contain a tumor cell expressing CD44v.

A further aspect of the present invention is a measurement method including the steps of simultaneously administering an aldehyde dehydrogenase inhibitor, or a compound (III) or a pharmacologically acceptable salt thereof, and a glutathione level reducer or a glutathione S-transferase inhibitor, to a tumor cell *in vitro;* and measuring a growth rate or a cell survival rate of the tumor cell:

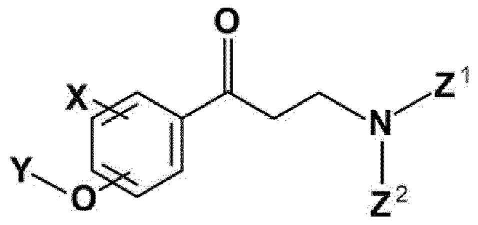

(III)

where X is hydrogen, halogen, $-NH_2$, or -CN, Y is a linear or branched C1-6 alkyl group, and $Z^1$ and $Z^2$ are hydrogen or halogen and a linear or branched C1-6 alkyl group optionally substituted with a substituent, respectively, the substituent being hydroxy or phenyl, or $Z^1$ and $Z^2$ form a 4-, 5-, 6-, or 7-membered azacycloalkyl group together with the neighboring nitrogen as a heteroatom.

In this measurement method, the tumor cell may be resistant to a glutathione level reducer or a glutathione S-transferase inhibitor.

A further aspect of the present invention is a method of identifying an agent that exerts a combined effect with a glutathione level reducer or a glutathione S-transferase inhibitor, including the steps of simultaneously administering a given glutathione level reducer or a given glutathione S-transferase inhibitor and each of a plurality of aldehyde dehydrogenase inhibitors, or compounds (III) or pharmacologically acceptable salts thereof, to a tumor cell *in vitro;* and measuring a growth rate or a cell survival rate of the tumor cell:

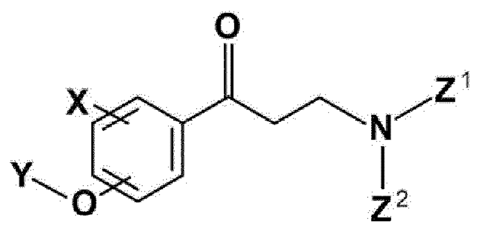

(III)

wherein X is hydrogen, halogen, $-NH_2$, or -CN, Y is a linear or branched C1-6 alkyl group, and $Z^1$ and $Z^2$ are hydrogen or halogen and a linear or branched C1-6 alkyl group optionally substituted with a substituent, respectively, the substituent being hydroxy or phenyl, or $Z^1$ and $Z^2$ form a 4-, 5-, 6-, or 7-membered azacycloalkyl group together with the neighboring nitrogen as a heteroatom.

A further aspect of the invention is a method for identifying a glutathione level reducer or a glutathione-S-transferase inhibitor that induces a combined effect with a compound (III) or a pharmacologically acceptable salt thereof, the compound (III) being an anti-tumor agent, the method including the steps of simultaneously administering the compound (III) and a plurality of glutathione level reducers or glutathione S-transferase inhibitors, to a tumor cell *in vitro;* and measuring a growth rate or a cell survival rate of the tumor cell:

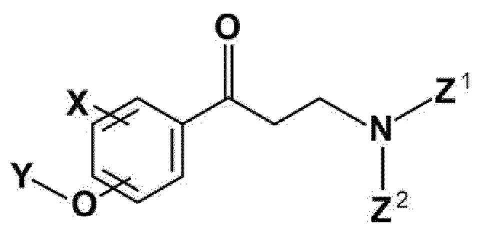

(III)

wherein X is hydrogen, halogen, $-NH_2$, or -CN, Y is a linear or branched C1-6 alkyl group, and $Z^1$ and $Z^2$ are hydrogen or halogen and a linear or branched C1-6 alkyl group optionally substituted with a substituent, respectively, the substituent being hydroxy or phenyl, or $Z^1$ and $Z^2$ form a 4-, 5-, 6-, or 7-membered azacycloalkyl group together with the neighboring nitrogen as a heteroatom.

The compound (III) may be a compound (II):

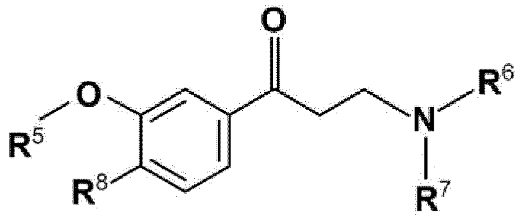

(I I)

wherein $R^5$ is a linear or branched C1-6 alkyl group, $R^2$ and $R^3$ are each independently selected from linear and branched C1-6 alkyl groups, or $R^2$ and $R^3$ form a 4-, 5-, 6-, or 7-membered azacycloalkyl group together with the neighboring nitrogen as a heteroatom, and $R^4$ is hydrogen or halogen.

A further aspect of the present invention is a method for identifying a tumor cell on which a compound (III) or a pharmacologically acceptable salt thereof and a glutathione level reducer or a glutathione S-transferase inhibitor have a combined effect, the compound (III) being an anti-tumor agent, the method including the steps of simultaneously administering a given combination of the compound (III) or a pharmacologically acceptable salt thereof and a glutathione level reducer or a glutathione S-transferase inhibitor, to different kinds of tumor cells *in vitro;* and measuring a growth rate or a cell survival rate of the different kinds of tumor cells.

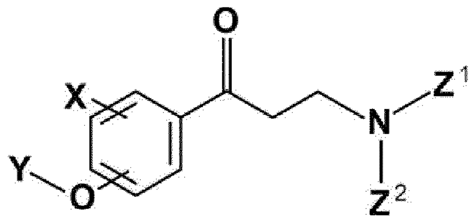

(I I I)

wherein X is hydrogen, halogen, $-NH_2$, or -CN, Y is a linear or branched C1-6 alkyl group, and $Z^1$ and $Z^2$ are hydrogen or halogen and a linear or branched C1-6 alkyl group optionally substituted with a substituent, respectively, the substituent being hydroxy or phenyl, or $Z^1$ and $Z^2$ form a 4-, 5-, 6-, or 7-membered azacycloalkyl group together with the neighboring nitrogen as a heteroatom.

The compound (III) may be a compound (II):

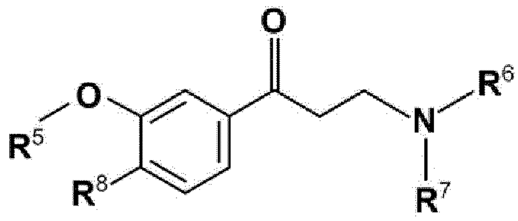

(I I)

wherein $R^5$ is a linear or branched C1-6 alkyl group, $R^2$ and $R^3$ are each independently selected from linear and branched C1-6 alkyl groups, or $R^2$ and $R^3$ form a 4-, 5-, 6-, or 7-membered azacycloalkyl group together with the neighboring nitrogen as a heteroatom, and $R^4$ is hydrogen or halogen.

In any one of the aforementioned identification method, the tumor cell may be resistant to a glutathione level reducer or a glutathione S-transferase inhibitor.

A further aspect of the present invention is an anti-tumor agent used in combination with radiation for radiotherapy, including, as an active ingredient, an aldehyde dehydrogenase inhibitor, or a compound (II) below or a pharmacologically acceptable salt thereof:

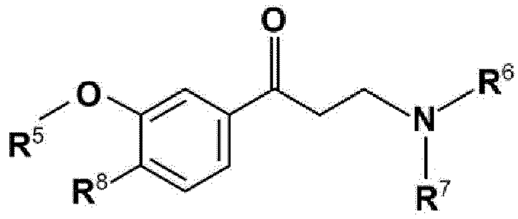

(II)

wherein $R^5$ is a linear or branched C1-6 alkyl group, $R^2$ and $R^3$ are each independently selected from linear and branched C1-6 alkyl groups, or $R^2$ and $R^3$ form a 4-, 5-, 6-, or 7-membered azacycloalkyl group together with the neighboring nitrogen as a heteroatom, and $R^4$ is hydrogen or halogen. The compound represented by the formula (II) may be oxyfedrine. The anti-tumor agent may be against a tumor containing a tumor cell resistant to a glutathione level reducer or a glutathione S-transferase inhibitor. The tumor cell may have a high expression of aldehyde dehydrogenase. The tumor may further contain a tumor cell expressing CD44v.

A further aspect of the present invention is a measurement method for an anti-tumor effect including the steps of exposing a tumor cell *in vitro* to a radiation in the presence of an aldehyde dehydrogenase inhibitor, or a compound (III) or a pharmacologically acceptable salt thereof; and measuring a growth rate or a cell survival rate of the tumor cell:

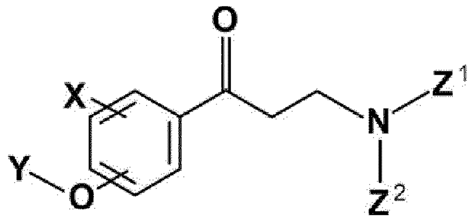

(III)

wherein X is hydrogen, halogen, $-NH_2$, or -CN, Y is a linear or branched C1-6 alkyl group, and $Z^1$ and $Z^2$ are hydrogen or halogen and a linear or branched C1-6 alkyl group optionally substituted with a substituent, respectively, the substituent being hydroxy or phenyl, or $Z^1$ and $Z^2$ form a 4-, 5-, 6-, or 7-membered azacycloalkyl group together with the neighboring nitrogen as a heteroatom. The tumor cell may be resistant to a glutathione level reducer or a glutathione S-transferase inhibitor.

A further aspect of the present invention is a method for identifying an agent that has a synergistic effect with irradiation in a tumor cell *in vitro,* the method including the steps of exposing a tumor cell *in vitro* to a radiation in the presence of an aldehyde dehydrogenase inhibitor, or each of more than one of compounds (III) or pharmacologically acceptable salts thereof; and measuring a growth rate or a cell survival rate of the tumor cell:

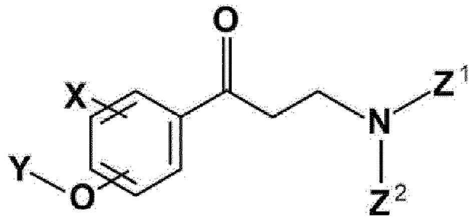

(III)

where X is hydrogen, halogen, $-NH_2$, or -CN, Y is a linear or branched C1-6 alkyl group, and $Z^1$ and $Z^2$ are hydrogen or halogen and a linear or branched C1-6 alkyl group optionally substituted with a substituent, respectively, the substituent being hydroxy or phenyl, or $Z^1$ and $Z^2$ form a 4-, 5-, 6-, or 7-membered azacycloalkyl group together with the neighboring nitrogen as a heteroatom. The compounds (III) may be a compound (II).

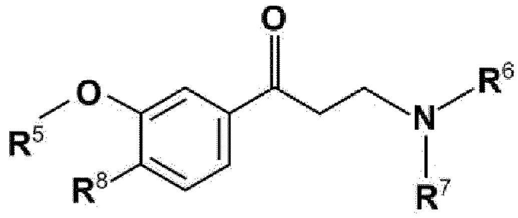

(I I)

where $R^5$ is a linear or branched C1-6 alkyl group, $R^2$ and $R^3$ are each independently selected from linear and branched C1-6 alkyl groups, or $R^2$ and $R^3$ form a 4-, 5-, 6-, or 7-membered azacycloalkyl group together with the neighboring nitrogen as a heteroatom, and $R^4$ is hydrogen or halogen.

The tumor cell may be resistant to a glutathione level reducer or a glutathione S-transferase inhibitor.

A further aspect of the present invention is an enhancer of an anti-tumor action by co-administration with an aldehyde dehydrogenase inhibitor, or a compound (II) below or a pharmacologically acceptable salt thereof, and a glutathione level reducer or a glutathione S-transferase inhibitor, the enhancer including a suppressor of an xCT expression-enhancing action of Nrf2:

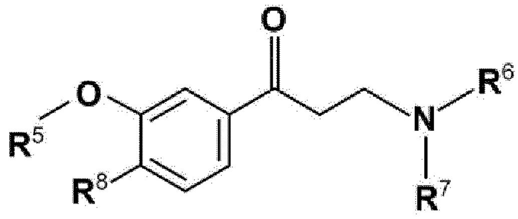

(I I)

wherein $R^5$ is a linear or branched C1-6 alkyl group, $R^6$ is hydrogen or halogen, $R^7$ is a linear or branched C1-6 alkyl group optionally substituted with a substituent, the substituent being hydroxy or phenyl, and $R^8$ is hydrogen or halogen. The suppressor may be an Nrf2-gene expression suppressing substance or an Nrf2 inhibitor. The Nrf2-gene expression suppressing substance may be an antisense NA, miNA, or siNA against an Nrf2 gene. The Nrf2 inhibitor may be ML385 or an anti-Nrf2 antibody. The anti-tumor action may be on a tumor overexpressing an Nrf2 gene. The glutathione level reducer may be sulfasalazine. The compound represented by the formula (II) may be oxyfedrine.

A further aspect of the present invention is a companion diagnostic drug for predicting an anti-tumor effect upon co-administration of an aldehyde dehydrogenase inhibitor, or a compound (II) below or a pharmacologically acceptable salt thereof, and a glutathione level reducer or a glutathione S-transferase inhibitor, the companion diagnostic drug including a detection reagent for detecting Nrf2 gene expression:

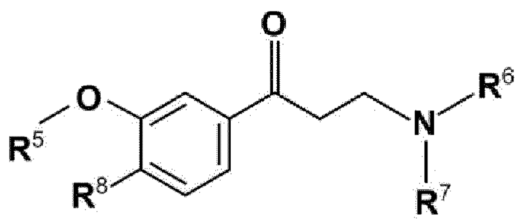

(I I)

wherein $R^5$ is a linear or branched C1-6 alkyl group, $R^6$ is hydrogen or halogen, $R^7$ is a linear or branched C1-6 alkyl group optionally substituted with a substituent, the substituent being hydroxy or phenyl, and $R^8$ is hydrogen or halogen. The detection reagent may be an antibody, a probe for detecting gene expression, or a primer for gene amplification. The glutathione level reducer may be sulfasalazine. The compound represented by the formula (II) may be oxyfedrine.

A further aspect of the present invention is a companion diagnostic drug for predicting an anti-tumor effect upon co-administration of an aldehyde dehydrogenase inhibitor, or a compound (II) below or a pharmacologically acceptable salt thereof, and a glutathione level reducer or a glutathione S-transferase inhibitor, the companion diagnostic drug including a detection reagent for detecting a mutation of Keap1 or Nrf2 gene:

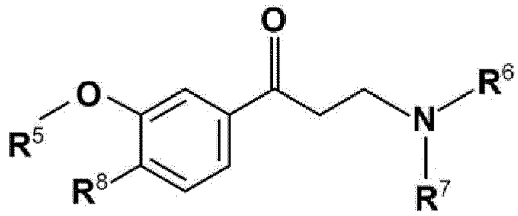

(I I)

wherein $R^5$ is a linear or branched C1-6 alkyl group, $R^6$ is hydrogen or halogen, $R^7$ is a linear or branched C1-6 alkyl group optionally substituted with a substituent, the substituent being hydroxy or phenyl, and $R^8$ is hydrogen or halogen.

==Cross-reference to related documents==

This application claims the priority of Japanese patent application 2019-091358 filed on May 14, 2019, and Japanese patent application 2019-200094 filed on November 1, 2019, which are hereby incorporated by reference in their entirety.

Brief description of the drawings

Fig. 1 illustrates a graph showing a combined effect of sulfasalazine and dyclonine in an example of the present invention.

Fig. 2 illustrates a graph illustrating changes in dyclonine sensitivity caused by xCT knockdown in an example of the present invention.

Fig. 3 illustrates a figure showing a combined effect of sulfasalazine, elastin, or BSO with dyclonine in various cancer cell lines in an example of the present invention.

Fig. 4 illustrates a graph showing a combined effect of sulfasalazine and dyclonine *in vivo* in an example of the present invention.

Fig. 5 illustrates a figure of experimental results showing an inhibitory effect of dyclonine on ALDH activity in an example of the present invention.

Fig. 6 illustrates a figure showing an effect on the accumulation of HNE (4-HNE; 4-hydroxy-2-nonenal) by combined use of sulfasalazine and dyclonine in an example of the present invention.

Fig. 7 illustrates graphs showing combined effects of sulfasalazine or BSO and dyclonine analogs (with a dyclonine backbone) in an example of the present invention.

Fig. 8 illustrates graphs showing combined effects of BSO and dyclonine analogs (without a dyclonine backbone) in an example of the present invention.

Fig. 9 illustrates graphs showing combined effects of sulfasalazine, elastin, or BSO and dyclonine on OSC19 cells or sulfasalazine-resistant OSC19 cells in an example of the present invention.

Fig. 10 illustrates graphs showing expressions of ALDH family genes in HSC4 cells, OSC19 cells, or sulfasalazine-resistant OSC19 cells in an example of the present invention.

Fig. 11 illustrates graphs of experimental results showing combined effects of oxyfedrine and sulfasalazine (SSZ) or L-buthionine-sulfoximine (BSO) in an example of the present invention.

Fig. 12 illustrates a diagram representing metabolic pathways of HNE Abbreviations: HNA, 4-hydroxy-2-nonenoic acid; GSH, glutathione; ALDH, aldehyde dehydrogenase; GST, glutathione S-transferase.

Fig. 13 illustrates a representation showing the reduction in the GSH levels in tumor cells by sulfasalazine or BSO in an example of the present invention.

Fig. 14 illustrates a representation showing combined effects of sulfasalazine or BSO and oxyfedrine on the cellular accumulation of HNE in an example of the present invention.

Fig. 15 illustrates graphs showing a combined effect of sulfasalazine and oxyfedrine on the reduction of the tumor cell viability *in vivo* in an example of the present invention.

Fig. 16 illustrates graphs showing a combined effect of sulfasalazine and oxyfedrine on the cellular accumulation of HNE *in vivo* in an example of the present invention.

Fig. 17 illustrates a representation of experimental results showing a combined effect of irradiation and the administration of oxyfedrine on the reduction of the tumor cell viability in an example of the present invention.

Fig. 18 illustrates a representation of experimental results showing a combined effect of irradiation and the administration of oxyfedrine on the cellular accumulation of HNE in an example of the present invention.

Fig. 19 illustrates a representation of experimental results showing Nrf2 and xCT expressions in SSZ-resistant tumor cells in an example of the present invention.

Fig. 20 illustrates a representation of experimental results showing cell viability of A549 cells in media containing siRNA against the Nrf2 gene or ML385 that is an inhibitor of Nrf2, OXY, and SSZ or BSO in an example of the present invention.

Fig. 21 illustrates a diagram of experimental results showing a combined effect of irradiation and the administration of oxyfedrine (OXY) on the suppression of weight gain of the tumor cells *in vivo* in an example of the present invention.

## Embodiments of the invention

Embodiments of the present invention are described in detail below with reference to examples. The objects, features, advantages, and ideas of the present invention are apparent to those skilled in the art from the description of this specification. Those skilled in the art can easily reproduce the present invention from the description herein. The embodiments and specific examples described below represent preferable aspects of the present invention given for the purpose of illustration or explanation, and are not construed to limit the present invention. It is obvious to those skilled in the art that various changes and modifications can be made based on the description of the present specification within the spirit and scope of the present invention disclosed herein.

Unless otherwise noted in an embodiment or an example, all procedures used are according to standard protocols, with or without modifications or changes. Commercial reagent kits and measurement instruments are used as described in protocols attached thereto, unless otherwise noted.

== Anti-tumor agent ==

An embodiment of the present invention is an anti-tumor agent comprising a glutathione level reducer as an active ingredient, the anti-tumor agent being administered simultaneously with an effective amount of an aldehyde dehydrogenase inhibitor or a compound (II) below or a pharmacologically acceptable salt thereof. As used herein, an "effective amount of an aldehyde dehydrogenase inhibitor" refers to the amount of an aldehyde dehydrogenase inhibitor that exerts a combined effect with a glutathione level reducer, as anti-tumor activity.

Another embodiment of the present invention is an anti-tumor agent comprising, as an active ingredient, an aldehyde dehydrogenase inhibitor or a compound (II) below or a pharmacologically acceptable salt thereof, the anti-tumor agent being administered simultaneously with an effective amount of a glutathione level reducer. As used herein, an "effective amount of a glutathione level reducer" refers to the amount of a glutathione level reducer that exerts a combined effect with an aldehyde dehydrogenase inhibitor, as anti-tumor activity.

While not bound by the following theory, as shown in Fig. 12, it is considered that cells have multiple pathways to metabolize HNE, and by simultaneous inhibition of GST- and ALDH-mediated pathways among these pathways, HNE accumulates in cells; then, since HNE has cytotoxicity, tumor cells cannot proliferate. Therefore, the simultaneous administration of an aldehyde dehydrogenase inhibitor, or a compound (II) below or a pharmacologically acceptable salt thereof and a glutathione level reducer exerts a synergistic anti-tumor effect.

Furthermore, another embodiment of the present invention is an anti-tumor agent used in radiotherapy, comprising, as an active ingredient, an aldehyde dehydrogenase inhibitor, or a compound (II) below or a pharmacologically acceptable salt thereof. As used herein, radiotherapy is a treatment method used to treat tumors. The radiation dosage and irradiation method can be readily assessed and determined by a practitioner according to the type of tumor and the patient's condition, based on common technical knowledge. It is known that the amount of intracellular GSH is decreased by irradiation, and thus irradiation in the presence of an aldehyde dehydrogenase inhibitor, or a compound (II) below or a pharmacologically acceptable salt thereof produces a combined effect of both. Accordingly, in this case, the anti-tumor agent is administered to a patient with a tumor and the patient may be irradiated while the anti-tumor agent is at a level at which the synergistic effect with irradiation is observed, or a patient with a tumor is irradiated and the anti-tumor agent may be administered to the patient while the GSH level is decreased.

The aldehyde dehydrogenase inhibitor is a drug that inhibits enzymatic activity of aldehyde dehydrogenase 2 (ALDH2) (EC 1.2.1.10). The types and isotypes of the targeted ALDH are not limited and may be any one of ALDH 1 to 5 and their isotypes. The aldehyde dehydrogenase inhibitor used in the anti-tumor agents is, for example, chlorpropamide, tolbutamide, diethylaminobenzaldehyde, disulfiram (tetraethylthioperoxydicarbonic diamide), cyanamide, oxyfedrine, citral (3,7-dimethyl-2,6-octadienal), coprine, daidzin, DEAB (4-(diethylamino)benzaldehyde), gossypol, kynurenine metabolites (3-hydroxykynurenine, 3-hydroxyanthranilic acid, kynurenic acid, and indol-3-ylpyruvic acid), molinate, nitroglycerin, purgiline (N-benzyl-N-methylprop-2-yn-1-amine) and analogs thereof, or pharmacologically acceptable salts thereof but is not limited thereto. In particular, the following dyclonine and dyclonine analogs (I) are preferred, and the compounds shown in Fig. 7 (BAS00363846, STL327701, PHAR033081, PHAR298639, and Aldi-2) are more preferred:

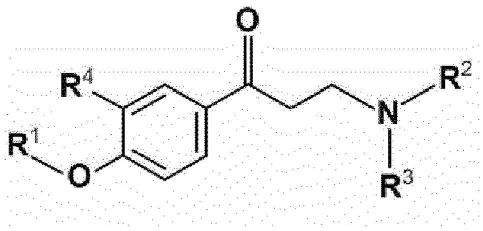

(I)

wherein $R^1$ is a linear or branched C1-6 alkyl group, $R^2$ and $R^3$ are each independently selected from linear and branched C1-6 alkyl groups, or $R^2$ and $R^3$ form a 4-, 5-, 6-, or 7-membered azacycloalkyl group together with the neighboring nitrogen atom as a heteroatom , and $R^4$ is hydrogen or halogen; $R^1$ is preferably a linear or branched C4-5 alkyl group, and $R^2$ and $R^3$ are preferably C2 alkyls, or $R^2$ and $R^3$ preferably form a 6-membered azacycloalkyl group together with the neighboring nitrogen atom as a heteroatom. It should be noted that the compound in which $R^1$ is a linear C4 alkyl and $R^2$ and $R^3$ form a 6-membered azacycloalkyl group together with the neighboring nitrogen atom is dyclonine. The halogen is preferably F, Cl, Br, or I.

The compound (II) is oxyfedrine or an analog thereof and has the following chemical formula:

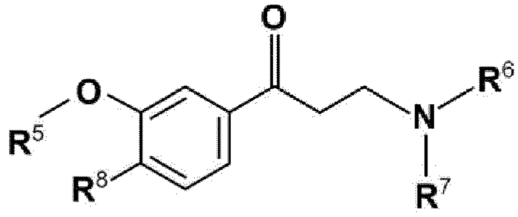

(I I)

wherein $R^5$ is a linear or branched C1-6 alkyl group, $R^6$ is hydrogen or halogen, $R^7$ is a linear or branched C1-6 alkyl group optionally substituted with a substituent, the substituent being hydroxy or phenyl, and $R^8$ is hydrogen or halogen.

The compound (II) to be used is preferably oxyfedrine having the following chemical formula (IV), and a salt thereof is preferably oxyfedrine hydrochloride.

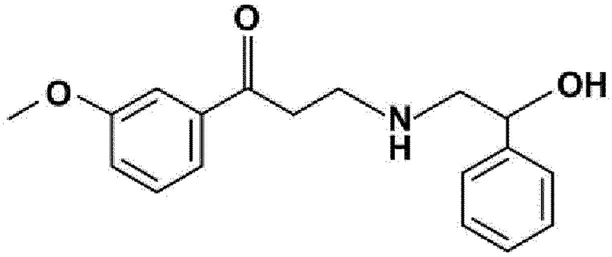

(I V)

The pharmacologically acceptable salts are not limited as long as they are formed with the above compounds. Specific examples include addition salts of inorganic acids such as hydrochloride, sulfate, nitrate, hydrobromide, hydriodide, perchlorate, and phosphate, addition salts of organic acids such as oxalate, maleate, fumarate, and succinate, addition salts of sulfonic acids such as methanesulfonate, ethanesulfonate, benzenesulfonate, *p*-toluenesulfonate, and camphor sulfonate, and addition salts of amino acids. The salt is preferably hydrochloride, oxalate, maleate, or methanesulfonate. Furthermore, it is a matter of course that those compounds or the pharmacologically acceptable salts thereof include anhydrides, as well as hydrates and crystal polymorphic forms.

The glutathione level reducer is a drug that reduce the cellular level of glutathione. Any glutathione level reducer may be used in the anti-tumor agents, but the glutathione level reducer is preferably a drug that inhibits the pathway through which glutathione is synthesized from cystine uptaken into the cells by xCT. More preferably, the glutathione level reducer is a drug that inhibits activity of xCT, thioredoxin-1 (TRX-1), glutamate-cysteine ligase (GCL) (EC 6.3.2.2) (also called $\gamma$-glutamylcysteine synthetase), and/or glutathione synthetase (EC 6.3.2.3), and yet more preferably, an xCT inhibitor or a GCL inhibitor. Any xCT inhibitor may be used, but the xCT inhibitor is preferably sulfasalazine, elastin,

sorafenib, or a derivative thereof, or an anti-xCT antibody. Likewise, any GCL inhibitor may be used, but the GCL inhibitor is preferably L-buthionine-sulfoximine or a derivative thereof. Any derivative, such as a PEGylated form, may be used as long as it can be a glutathione level reducer.

The glutathione S-transferase inhibitor is a drug that inhibits enzymatic activity of glutathione S-transferase (EC 2.5.1.18), and in particular a drug that inhibits the activity of converting HNE (4-HNE; 4-hydroxy-2-nonenal) to HNE-GSH. Any glutathione S-transferase inhibitor may be used, and examples include glutathione analogs (e.g., WO95/08563, WO96/40205, and WO99/54346), ketoprofen, indomethacin, ethacrynic acid, piriprost, anti-GST antibodies, and GST dominant-negative mutants.

As used herein, "simultaneously administering" two or more drugs refers to administering these drugs at the same time, and also administering them independently at different times, as long as one drug is administered while the preceding drug(s) is/are effective. When two or more drugs are administered simultaneously, two or more different agents, each containing one of the drugs, may be administered at the same time, or two or more drugs may be administered in a single dosage form as a combination drug. The terms "co-administration" and "co-administering" also have the same meaning as "simultaneous administration" and "simultaneously administering," respectively.

The subject to which an anti-tumor agent is administered may be any vertebrate, but it is preferably a human cancer patient. The tumor to be treated may be any tumor, but is preferably a tumor containing tumor cells resistant to a glutathione level reducer or a glutathione S-transferase inhibitor. In the tumor cells, aldehyde dehydrogenase may be expressed at a high level. The glutathione level reducer or the glutathione S-transferase inhibitor is preferably an xCT inhibitor, and is more preferably sulfasalazine. The tumor cell resistant to a drug refers to a tumor cell that survives when the drug is administered to a patient at an ordinary therapeutic level and for an ordinary number of therapeutic days in vivo or a tumor cell that survives at a survival rate of 90% or more when exposed to the drug at a level corresponding to the cell viability of 50% or less in 80% or more kinds of cell lines. For example, a sulfasalazine-resistant tumor cell refers to a tumor cell that survives when sulfasalazine is administered to a patient at an $AUC_{0-24}$ of 50-300 μg·h/mL for approximately 2 weeks *in vivo,* and a tumor cell that has a survival rate of 90% or more at 200 μM. *in vitro.* Likewise, an "L-buthionine-sulfoximine-resistant cell" refers to a tumor cell that survives when L-buthionine-sulfoximine is administered to a patient at an $AUC_{0-24}$ of 10-100 μg·h/mL for approximately 2 weeks *in vivo,* and a tumor cell that has a survival rate of 90% or more at 100 μM *in vitro.* The CD44v expression level in sulfasalazine-resistant tumor cells and L-buthionine-sulfoximine-resistant cells preferably is low or negative. The tumor cell in which aldehyde dehydrogenase is overexpressed refers to a cell in which the ALDH1A1, ALDH2, ALDH1B1, or ALDH3A1 gene is expressed at a level that is at least threefold, preferably tenfold, higher compared with OSC19 cells. In the tumor to be treated, tumor cells expressing CD44v may be contained, because sulfasalazine and L-buthionine-sulfoximine has an efficient anti-tumor function on tumor cells expressing CD44v. The "tumor cells expressing CD44v" may be any cells in which CD44v expression can be detected, but are preferably cells with a high level of CD44v expression. The high level in such cases means a level equal to or higher than the average level in ovarian tumor cells, but the level is preferably 2-fold or higher, more preferably 4-fold or higher, and yet more preferably 10-fold or higher.

Tumors herein may be of any type, but are preferably solid cancers. Examples include colorectal adenocarcinoma, gastric adenocarcinoma, breast adenocarcinoma, lung adenocarcinoma, pancreatic adenocarcinoma, squamous cell carcinoma of the head and neck, ovarian tumor, and testicular tumor.

The anti-tumor agent can be formulated into dosage forms such as tablets, fine powders, granules, powders, capsules, syrups, emulsions, and suspending agents by an ordinary method. The anti-tumor agents are produced using a pharmaceutically acceptable additive known to those skilled in the art, such as an excipient and a carrier.

The anti-tumor agent can be administered to the subject in a range of effective amount via a suitable route. The effective amount can be appropriately determined by a physician or a veterinarian in consideration of, for example, the dosage form, administration route, age and weight of the subject, and disease conditions of the subject. By way of example, the dose of a compound is preferably 0.1 mg/kg/day or more, more preferably 1 mg/kg/day or more, and yet more preferably 10 mg/kg/day. The dose is preferably 1000 mg/kg/day or less, more preferably 300 mg/kg/day or less, and yet more preferably 100 mg/kg/day or less. Any administration method may be used. For example, the compound may be administered orally or parenterally by intraperitoneal or intravenous injection or infusion, or injected directly into a tumor.

== Enhancers potentiating anti-tumor actions of anti-tumor agents ==

One embodiment of the present invention is an enhancer potentiating the anti-tumor action caused by co-administration with an aldehyde dehydrogenase inhibitor, or a compound (II) below or a pharmacologically acceptable salt thereof, and a glutathione level reducer or a glutathione S-transferase inhibitor, the enhancer comprising a suppressor of an xCT expression-enhancing function of Nrf2. The enhancer can be said an enhancer potentiating the anti-tumor action of the aforementioned anti-tumor agents. The enhancer potentiating the anti-tumor action of the anti-tumor agents is administered simultaneously with the anti-tumor agents.

As shown in the Example, the Nrf2 expression level is positively correlated with the xCT and ALDH expression levels. While not bound by this theory, it is considered that suppressing the Nrf2 expression level leads to suppressing the expression of xCT and ALDH and potentiating the efficacy of anti-tumor agents.

Examples of the suppressor of an xCT expression-enhancing action of Nrf2 include Nrf2-gene expression suppressing substances and inhibitors of an xCT expression-enhancing function of Nrf2. Specifically, in order to suppress the xCT expression-enhancing action of Nrf2, the Nrf2 gene expression may be suppressed in the tumor cells, or the xCT expression-enhancing function of Nrf2 as a protein may be inhibited.

Examples of the Nrf2-gene expression suppressing substance include antisense NA, miNA, or siNA against the Nrf2 gene. Each may consist of RNA, or DNA, or be a chimeric molecule of RNA and DNA. The nucleic acids (NAs) may also have various modifications. Their sequences can be easily designed from the technical knowledge of those skilled in the art. Examples of the Nrf2 inhibitor includes small-molecule compounds such as ML385 and anti-Nrf2 antibodies.

Although administration target may be any tumor cells, the administration target is preferably a tumor overexpressing the Nrf2 gene because tumors overexpressing the Nrf2 gene are resistant to the aforementioned anti-tumor agent(s). Accordingly, the expression level of the Nrf2 gene may be examined in the tumor cells as an administration target, prior to the administration of an enhancer potentiating the anti-tumor action of an anti-tumor agent. If the expression level of the Nrf2 gene is normal, an anti-tumor agent alone may be administered, and an enhancer potentiating the anti-tumor action of that anti-tumor agent may also be administered simultaneously; if the level of the Nrf2 gene expression is higher than normal, it is preferable to co-administer an anti-tumor agent and an enhancer potentiating the anti-tumor action of that anti-tumor agent.

== Measurement method for a growth rate or a cell survival rate of tumor cells ==

One embodiment of the present invention is a measurement method, comprising the steps of simultaneously administering an aldehyde dehydrogenase inhibitor, or a compound (III) or a pharmacologically acceptable salt thereof, and a glutathione level reducer or a glutathione S-transferase inhibitor, to tumor cells *in vitro;* and measuring a growth rate or a cell survival rate of the tumor cells to which the drugs have been administered.

Another embodiment of the present invention is a measurement method comprising the steps of exposing tumor cells *in vitro* to a radiation in the presence of an aldehyde dehydrogenase inhibitor, or a compound (III) or a pharmacologically acceptable salt thereof; and measuring a growth rate or a cell survival rate of the tumor cells.

For the aldehyde dehydrogenase inhibitors, the glutathione level reducers, and the glutathione S-transferase inhibitors in this section, it is possible to refer to those described in detail in the "Anti-tumor agents" section. The compound (III) has the following chemical formula, but is preferably the compound (II).

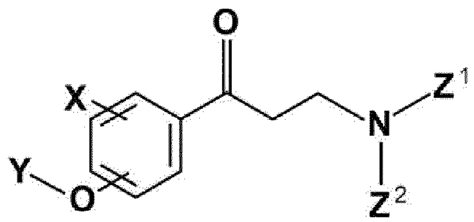

(III)

wherein X is hydrogen, halogen, $-NH_2$, or -CN, Y is a linear or branched C1-6 alkyl group, and $Z^1$ and $Z^2$ are hydrogen or halogen and a linear or branched C1-6 alkyl group optionally substituted with a substituent, respectively, the substituent being hydroxy or phenyl, or $Z^1$ and $Z^2$ form a 4-, 5-, 6-, or 7-membered azacycloalkyl group together with the neighboring nitrogen as a heteroatom.

Since the aldehyde dehydrogenase inhibitor, or the compound (III) or a pharmacologically acceptable salt thereof, and the glutathione level reducer or the glutathione S-transferase inhibitor have cooperative anti-tumor activity, this measuring method can be used to identify combinations of drugs that induce their effects in a cooperative manner or to find a set of drugs that are highly effective when used in combination, or to find tumor cells on which a certain combination of drugs works quite effectively.

Specifically, an aldehyde dehydrogenase inhibitor, or a compound (III) or a pharmacologically acceptable salt thereof that exerts a combined effect with a given glutathione level reducer or a given glutathione S-transferase inhibitor can be identified by simultaneously administering the given glutathione level reducer or the given glutathione S-transferase inhibitor and each of a plurality of aldehyde dehydrogenase inhibitors, or compounds (III) or pharmacologically acceptable salts thereof, to tumor cells *in vitro,* and measuring a growth rate or a cell survival rate of the tumor cells to

which the drugs have been administered. Likewise, a glutathione level reducer or a glutathione S-transferase inhibitor that exerts a combined effect with a given aldehyde dehydrogenase inhibitor, or a given compound (III) or a pharmacologically acceptable salt thereof can be identified by simultaneously administering the given aldehyde dehydrogenase inhibitor, or the given compound (III) or a pharmacologically acceptable salt thereof and each of a plurality of glutathione level reducers or glutathione S-transferase inhibitors, to tumor cells *in vitro,* and measuring a growth rate or a cell survival rate of the tumor cells to which the drugs have been administered. Or, a drug that has a synergistic effect with irradiation can be identified by irradiating tumor cells *in vitro* in the presence of an aldehyde dehydrogenase inhibitor, or each of a plurality of compounds (III) or pharmacologically acceptable salts thereof; and measuring a growth rate or a cell survival rate of the tumor cells.

Furthermore, tumor cells on which an aldehyde dehydrogenase inhibitor, or a compound (III) or a pharmacologically acceptable salt thereof and a glutathione level reducer or a glutathione S-transferase inhibitor have a combined effect can be identified by simultaneously administering a given combination of an aldehyde dehydrogenase inhibitor, or a compound (III) or a pharmacologically acceptable salt thereof and a glutathione level reducer or a glutathione S-transferase inhibitor, to a plurality of tumor cell types *in vitro,* and measuring a growth rate or a cell survival rate of the tumor cells to which the drugs have been administered. The compound (III) used in these methods is preferably an anti-tumor agent that has anti-tumor activity.

== Companion diagnostic drug ==

An embodiment of the present invention is a companion diagnostic drug for predicting the anti-tumor effect of the aforementioned anti-tumor agents, and includes a reagent for detecting Nrf2 gene expression.

In recent years, Nrf2 expression has been known to be a factor of malignancy progression of tumor. As demonstrated in the Examples, anti-tumor agents are relatively less effective against tumor cells with high levels of Nrf2 expression. While not bound by this theory, it is considered that since Nrf2 expression levels are positively correlated with the xCT and ALDH expression levels, and the aforementioned anti-tumor agents simultaneously suppress the xCT and ALDH expressions, anti-tumor agents are less effective against cells with high levels of Nrf2 expression. Therefore, it is anticipated that the higher the level of the Nrf2 gene expression, the less effective the anti-tumor agent and that the lower the level of the Nrf2 gene expression, the more effective the anti-tumor agent.

Nrf2 gene expression can be detected at any stage to the final product of Nrf2 protein. For example, its mRNA or protein may be detected. Reagents for detecting the Nrf2 gene expression are not limited and can be easily selected according to common technical knowledge; they may include an antibody, a probe for detecting gene expression, or primers for gene amplification. A person skilled in the art can readily generate anti-Nrf2 antibodies and design probes for detection of gene expression and primers for gene amplification according to common technical knowledge.

Since mutations in Keap1 and Nrf2 genes are occasionally involved in the constitutive expression of the Nrf2 protein, companion diagnostic drugs also serve as reagents for detection of known mutations in Keap1 or Nrf2 genes. Mutations in Keap1 or Nrf2 genes can be detected using a known technique. Primers for gene amplification to amplify the Keap1 or Nrf2 genes may be included.

## Examples

(Experimental Example 1) Combined effects of sulfasalazine and dyclonine

(Purpose)

This experimental example shows that sulfasalazine and dyclonine, which have xCT inhibitory effects, have a combined effect on the reduction of the viability of sulfasalazine-resistant cells.

(Methods)

HSC-4, a sulfasalazine-resistant oral squamous carcinoma cell line, was seeded in a 96-well plate at 2000 cells/well, and culture was started. DMEM was used as the culture medium. After 24 hours, the medium was replaced with a medium containing 50 $\mu$M dyclonine or an equal volume of DMSO, as well as 0 (not added), 50, 100, 200, or 400 $\mu$M sulfasalazine, and the culture was continued for 48 hours. Then, the cells were assayed for cell viability using CellTiter-Glo (Promega), and cell survival rates in each case was calculated taking the number of live cells in the control (DMSO added, no sulfasalazine added) as 100%. A graph showing the survival rates at the indicated concentrations of sulfasalazine is presented in Fig. 1.

(Results)

HSC4 is a sulfasalazine-resistant cell line, and treatment with sulfasalazine alone has almost no effect on cell survival. Treatment with dyclonine alone (dyclonine added; no sulfasalazine added) results in 80% cell survival. However, when both dyclonine and sulfasalazine are added, the cell survival is reduced to 10% or less at 100 μM or more of sulfasalazine.

Thus, sulfasalazine and dyclonine have a combined effect on the reduction of the sulfasalazine-resistant cell survival.

(Experimental Example 2) Changes in dyclonine sensitivity caused by xCT knockdown

(Purpose)

In this experimental example, it is shown that by doing xCT knockdown instead of using sulfasalazine with an xCT inhibitory effect, the similar combined effect with dyclonine are obtained, thereby showing that the combined effect of sulfasalazine and dyclonine is mediated by the xCT inhibitory effect of sulfasalazine.

(Methods)

HSC-4 cells, a sulfasalazine-resistant oral squamous carcinoma cell line, were seeded in a 96-well plate at 3000 cells/well, and nonsilencing control (scrambled (Sense: UUCUCCGAACGUGUCACGUtt (SEQ ID NO. 1), Antisense: ACGUGACACGUUCGGAGAAtt (SEQ ID NO. 2))) and siRNA or xCT specific siRNA (xCT siRNA#1 Sense: AGAAAUCUGGAGGUCAUUAtt (SEQ ID NO. 3), Antisense: AGAAAUCUGGAGGUCAUUAtt (SEQ ID NO. 4), xCT siRNA#2 Sense: CCAGAACAUUACAAAUAAUtt (SEQ ID NO. 5), Antisense: AUUAUUUGUAAUGUUCUGGtt (SEQ ID NO. 6)) were lipofected using Lipofectamine RNAiMAX (Thermo Fisher Scientific), and culture was started. DMEM was used as the culture medium. After 24 hours, the medium was replaced with a medium containing 50 μM dyclonine (solvent: DMSO) or an equal volume of DMSO, and the culture was continued for 48 hours. Then, the cells were assayed for cell viability using CellTiter-Glo (Promega), and cell survival rates in each case were calculated taking the cell survival rate in the control (nonsilencing control, DMSO added) as 100%. The results are presented in Fig. 2.

(Results)

Treatment with 50 μM dyclonine alone results in HSC-4 cell survival rate of approximately 60%, whereas xCT knockdown in the presence of 50 μM dyclonine reduces the cell survival rate to only about 10-20%.

Thus, the combined effect of sulfasalazine and dyclonine is mediated by the xCT inhibitory effect of sulfasalazine.

(Experimental Example 3) Combined effects of sulfasalazine, elastin or BSO with dyclonine in various tumor cell lines

(Purpose)

In this experimental example, it is shown that sulfasalazine, a specific xCT inhibitor elastin, or an inhibitor of glutathione synthesis inhibitor BSO exerts a combined effect with dyclonine on various tumor cell lines. It is also shown that the inhibition of xCT is mediated by the inhibition of glutathione synthesis.

(Methods)

The cell lines presented in Fig. 3 were seeded in a 96-well plate at 3000 cells/well, and culture was started. DMEM was used as the culture medium. After 24 hours, the medium was replaced with a medium containing 50 μM dyclonine or an equal volume of DMSO, as well as 0 (not added) or 400 μM sulfasalazine, 0 (not added) or 5 μM elastin, or 0 (not added) or 100 μM BSO, and the culture was continued for 48 hours. Then, the cells were assayed for cell viability using CellTiter-Glo (Promega), and a cell survival rate in each case was calculated taking the number of live cells in the control (DMSO added, no dyclonine added) as 100%. The survival rate in each case is depicted in Fig. 3.

(Results)

Similar combined effects were observed when sulfasalazine, elastin, or BSO was combined with dyclonine, although the level of this effect varies depending on the cell type.

Thus, the xCT inhibition by sulfasalazine or elastin exerts its anti-tumor effect by inhibiting glutathione synthesis.

Therefore, a glutathione level reducer or a glutathione S-transferase inhibitor can be used instead of sulfasalazine or elastin.

(Experimental Example 4) *In vivo* combined effects of sulfasalazine and dyclonine

(Purpose)

In this experimental example, it is shown that the combined effects of sulfasalazine and dyclonine can be observed *in vivo*.

(Methods)

$1 \times 10^6$ cells of sulfasalazine-resistant, oral squamous carcinoma cell line HSC-2 were subcutaneously transplanted in nude mice. On Day 4 and the following consecutive days to Day 22 after transplantation, the mice were injected intraperitoneally with physiological saline, sulfasalazine alone, dyclonine alone, or a combination of sulfasalazine and dyclonine once a day, at a dose of 400 mg/kg and 5 mg/kg, respectively. The major and minor tumor axes were measured every 3 to 4 days, and the tumor volumes were calculated using the following equation. The results are plotted as presented in Fig. 4.

$$\text{Tumor volume} = (\text{major axis} \times (\text{minor axis})^2)/2$$

The tumor volumes were statistically analyzed using Student's *t*-test on Day 22.

(Results)

As shown in Fig. 4, treatment with each drug alone reduced tumor volume by approximately 35%, whereas administration of both agents reduced the volume by approximately 70%.

Thus, administration of sulfasalazine in combination with dyclonine can suppress the growth of sulfasalazine-resistant tumors.

(Experimental Example 5) Inhibition of ALDH by dyclonine

(Purpose)

The purpose of this experimental example is to demonstrate that dyclonine induces an inhibitory activity on ALDH.

(Methods)

HSC-4 cells, a sulfasalazine-resistant oral squamous carcinoma cell line, were seeded at $8 \times 10^5$ cells/dish in 10-cm cell culture dishes, and culture was started. DMEM was used as the culture medium. After 24 hours, the medium was replaced with a medium containing 50 $\mu$M dyclonine (solvent: DMSO), and the cells were cultured for another 24 hours. The cells were then collected, stained with the ALDEFLUOR Kit (STEMCELL Technologies) for ALDH activity in the presence of N,N-diethylaminobenzaldehyde (DEAB), and analyzed by FACS ("Dyclonine" in the figure). As a control, experimental results are shown for cells cultured in the absence of DEAB which were not stained with the ALDEFLUOR Kit ("Unstained" in the figure), and cells that were stained with the ALDEFLUOR Kit after replacing the medium with an equal volume of medium with DMSO and without dyclonine ("Non-treatment" in the figure). For the positive cell counts, a gate was set to gate out positive cells to almost 0% in the DMSO-treated sample stained with the ALDEFLUOR Kit in the presence of DEAB ("DEAB" in the figure), and the positive rate in each case was calculated.

(Results)

As shown in Fig. 5, the cell population with high ALDH activity was approximately 25% in the DMSO-treated cells, whereas that with high ALDH activity is reduced to approximately 1% in the dyclonine-treated cells and in the cells treated with DEAB, which is a known ALDH inhibitor.

Thus, dyclonine has an inhibitory activity on ALDH.

(Experimental Example 6) Accumulation of HNE by combined treatment with sulfasalazine and dyclonine

(Purpose)

In this experimental example, it is shown that combined treatment with sulfasalazine and dyclonine markedly increases the HNE level in tumor cells and the frequency of HNE-accumulating cells.

(Methods)

As in Experimental Example 1, HSC-4 cells were cultured in a medium containing 50 μM dyclonine or an equal volume of DMSO and 0 μM (not added) or 400 μM sulfasalazine, and the treated cells were fixed with 4% PFA-PBS. Then, the cells were permeabilized with 0.2% Triton X100-PBS, followed by blocking with 3% BSA-PBS. Subsequently, fluorescent staining was performed using an anti-HNE antibody as the primary antibody and AlexaFluor 488-conjugated anti-mouse IgG antibody as the secondary antibody. As a positive control, cells incubated with 50 μM HNE for 30 min were used and stained using antibodies in a similar manner. The images observed via fluorescence microscopy are presented in Fig. 6.

(Results)

When cells were treated with dyclonine or sulfasalazine alone, an increase in intracellular HNE level was observed at a low frequency, but when sulfasalazine and dyclonine were used in combination, an accumulation of intracellular HNE at a high frequency and at a high level was observed.

Thus, the combined use of xCT and ALDH inhibitors makes intracellular HNE accumulation detectable at a high frequency and at a high level. As the mechanism, it is considered that since cells have multiple pathways to metabolize HNE (see Fig. 12), simultaneous inhibition of both GST- and ALDH-mediated pathways causes HNE accumulation in the cells. It is also considered that tumor cells cannot proliferate due to HNE cytotoxicity.

(Experimental Example 7) Combined effects of sulfasalazine or BSO with dyclonine analogs (those with a dyclonine backbone)

(Purpose)

It is shown that the following dyclonine analogs (I) with a dyclonine backbone exerts a combined effect with sulfasalazine or BSO.

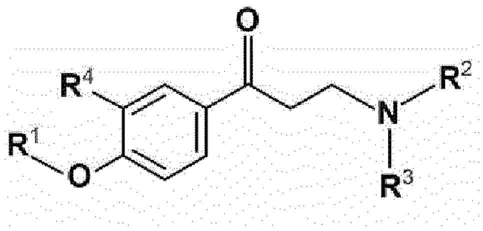

(I)

wherein $R^1$ is a linear or branched C1-6 alkyl group, $R^2$ and $R^3$ are each independently selected from linear and branched C1-6 alkyl groups, or $R^2$ and $R^3$ form a 4-, 5-, 6-, or 7-membered azacycloalkyl group together with the neighboring nitrogen as a heteroatom, and $R^4$ is hydrogen or halogen; $R^1$ is preferably a linear or branched C4-5 alkyl group, and $R^2$ and $R^3$ are preferably a C2 alkyl group or $R^2$ and $R^3$ preferably form a 6-membered azacycloalkyl group together with nitrogen as a heteroatom. It should be noted that the compound in which $R^1$ is a linear C4 alkyl group and $R^2$ and $R^3$ forms a 6-membered azacycloalkyl group together with the neighboring nitrogen as a heteroatom is dyclonine. The halogens are preferably F, Cl, I, Br, or I.

(Methods)

Similar to Experimental Example 1, HSC-4 cells were cultured in a medium containing 0 (not added), 25, 50, or 100 μM dyclonine, or 12.5, 25, 50, or 100 μM dyclonine analog BAS00363846, STL327701, PHAR033081, PHAR298639, or Aldi-2 (see Figure 7B regarding their chemical structures), and 0 (not added) or 100 μM BMBSO or

300 μM sulfasalazine; subsequently, the cells were assayed for cell viability. The results were plotted as presented in Fig. 7A.

(Results)

All compounds had a combined effect with BSO or sulfasalazine.

Thus, dyclonine analogs (I) with a dyclonine backbone exert combined effects as xCT inhibitors and anti-tumor agents.

(Experimental Example 8) Combined effects of BSO and dyclonine analogs (without a dyclonine backbone)

(Purpose)

It is shown that dyclonine analogs without a dyclonine backbone do not exert a combined effect with BSO.

(Methods)

Similar to Experimental Example 1, HSC-4 cells were cultured in a medium containing 0 (not added), 12.5, 25, or 50 μM dyclonine, or 3.125, 6.25, 12.5, 25, 50, or 100 μM dyclonine analog (4-hydroxyacetpphenone: see Fig. 8B regarding its chemical structure), and 0 (not added) or 100 μM BSO; subsequently, the cells were assayed for cell viability. The results are plotted as presented in Fig. 8A.

(Results)

Dyclonine analogs without a dyclonine backbone did not have any combined effect with BSO.

Thus, the dyclonine backbone is important for interacting with xCT inhibitors.

(Experimental Example 9) Combined effects of dyclonine with sulfasalazine, elastin, or BSO on sulfasalazine-resistant OSCC19 cells

(Purpose)

It is shown that dyclonine exerts a combined effect with glutathione synthesis inhibitors on cancer cell lines that have acquired resistance to xCT inhibitors.

Sulfasalazine-sensitive, oral squamous carcinoma cell line OSC19 was cultured for 2 months in a DMEM medium containing sulfasalazine to establish sulfasalazine-resistant OSC19 cells. The parental OSC19 and OSC19-SSZR cells were seeded in 96-well plates at 3000 cells/well. After 24 hours of culture, each medium was replaced with a medium containing sulfasalazine, elastin, or BSO at the concentrations indicated in Fig. 9, as well as 50 μM dyclonine (solvent: DMSO) or an equal volume of DMSO. The cells were cultured for 48 hours. Then, the cells were assayed for cell viability using CellTiter-Glo (Promega), and a cell survival rate in each case was calculated taking the cell survival rate in the control (no sulfasalazine, elastin, and BSO; DMSO added) as 100%.

(Results)

Dyclonine also exerted a combined effect with sulfasalazine, elastin or BSO on OSC19-SSZR cells.

Thus, dyclonine exerts a combined effect with glutathione synthesis inhibitors on cancer cell lines that have acquired resistance to xCT inhibitors.

(Experimental Example 10) ALDH family gene expression in sulfasalazine resistant OSC19 cells and HSC-4

(Purpose)

It is shown that cancer cells resistant to xCT inhibitors have a high expression level of ALDH family genes.

(Methods)

Messenger RNA was extracted from HSC-4, OSC19, and OSC19-SSZR cells, and complementary DNA was synthesized via reverse transcription. The expression levels of ALDH1A1, ALDH1B1, ALDH2, ALDH3A1, and RPS17

were measured by quantitative RT-PCR using the obtained complementary DNAs as template. The expression levels of each ALDH family gene were quantified by the ΔΔCt method using the expression level of RPS17 as a reference, and the results were graphically presented in Fig. 10.

(Results)

ALDH1A1 was expressed at a higher level in OSC19-SSZR cells than in OSC19 cells. Furthermore, ALDH1B1 and ALDH2 were highly expressed in HSC4, and ALDH3A1 was highly expressed in HSC4 and OSC19-SSZR. Thus, the expression levels of the ALDH family genes tended to be higher in xCT low-sensitive cancer cell lines.

In cancer cells with high expressions of ALDH family genes, HNE is metabolized by ALDH family genes. Therefore, even when the metabolism to GST is suppressed by an xCT inhibitor, toxicity of HNE is not effective, and cells acquire resistance to xCT inhibitors (see Fig. 12). Since the administration of ALDH inhibitors to such cells enhances their sensitivity to xCT inhibitors, anti-tumor agents containing an ALDH inhibitor and a glutathione level reducer or a glutathione S-transferase inhibitor are effective against cancer cells with high expression of ALDH family genes.

(Experimental Example 11) Combined effects of oxyfedrine (OXY) with sulfasalazine (SSZ) or L-buthionine-sulfoximine (BSO)

(Purpose)

It is shown to demonstrate that sulfasalazine or L-buthionine-sulfoximine and oxyfedrine have a combined effect on the reduction of the viability of sulfasalazine- or L-buthionine-sulfoximine-resistant tumor cells (A549, HCT116, and HSC-4).

(Methods)

Alveolar basal epithelial adenocarcinoma cell line A549, colon adenocarcinoma cell line HCT116, and oral squamous carcinoma cell lineHSC-4 , all of which are resistant to sulfasalazine or L-buthionine-sulfoximine, were seeded in 96-well plates at 4000 cells/well, and the culture was started. RPMI was used for the A549 and DMEM was used for the HCT116 and HSC-4 as culture media. After 24 hours, each medium was replaced with a medium containing 50 or 100 μM oxyfedrine or an equal volume of DMSO, and 0 (no sulfasalazine and no L-buthionine sulfoximine) or 400 μM sulfasalazine or 100 μM L-buthionine sulfoximine, and the culture was continued for 48 hours. Then, the cells were assayed for cell viability using CellTiter-Glo (Promega), and a cell survival rate in each case was calculated taking the number of live cells in the control (DMSO added, and sulfasalazine and L-buthionine sulfoximine not added) as 100%. A graph illustrating the survival at the indicated concentrations of oxyfedrine is presented in Fig. 11.

(Results)

A549, HCT116 and HSC4 are sulfasalazine- and/or buthionine sulfoximine-resistant cell lines, and treatment with sulfasalazine or buthionine sulfoximine alone has little effect on cell survival rate. In addition, treatment with 50 μM oxyfedrine alone (with oxyfedrine and without sulfasalazine) has no significant cytotoxic effect. When both oxyfedrine and sulfasalazine or buthionine sulfoximine are added, however, cell survival rate was reduced to 25% or less and 5% or less in the presence of sulfasalazine and buthionine sulfoximine, respectively, with, for example, 100 μM oxyfedrine .

Thus, sulfasalazine or buthionine sulfoximine and oxyfedrine have a synergistic combined effect on the reduction of the sulfasalazine-resistant cell survival rate. The level of this effect somewhat varies depending on the cell type. For example, a lower level is preferred when considering its administration to patients. The cell survival rate as a combined effect of sulfasalazine with 50 μM oxyfedrine is almost identical to that of sulfasalazine alone in A549 cells, whereas it is 75% in both HCT116 and HSC4 cells; thus, the combined effect of oxyfedrine and sulfasalazine is weaker in A549 compared with that in HCT116 and HSC-4 cells.

(Experimental Example 12) Reduction in the GSH levels in tumor cells by sulfasalazine (SSZ) or buthionine sulfoximine (BSO)

(Purpose)

In this experimental example, it is shown that the GSH levels in tumor cells are reduced by SSZ or BSO.

(Methods)

SSZ-resistant tumor cells (HCT116 and HSC-4) were used and the cells were treated in a similar manner to that in Example 11, and the intracellular GSH levels were measured after 48 hours using a GSH-Glo Glutathione Assay Kit (Promega). The measurement results are presented in Fig. 13.

(Results)

When the cells are treated with SSZ, BSO, or oxyfedrine (OXY) alone, the intracellular GSH levels were reduced by SSZ or BSO alone, but the effect of SSZ alone on the reduction of the intracellular GSH levels was not much strong. Treatments with OXY alone did not reduce the intracellular GSH levels. On the contrary, when SSZ and OXY were used in combination, the intracellular GSH levels were reduced more than SSZ alone was used.

Thus, SSZ or BSO acts as an xCT inhibitor and reduces GSH levels in tumor cells, but the effect of SSZ alone is not sufficient.

(Experimental Example 13) Accumulation of HNE due to the combined use of sulfasalazine (SSZ) or buthionine sulfoximine (BSO) with oxyfedrine (OXY)

(Purpose)

In this experimental example, it is shown that combined treatment with SSZ or BSO and OXY markedly increases the HNE level in tumor cells as well as the frequency of HNE-accumulating cells.

(Methods)

In this experimental example, sulfasalazine-resistant tumor cells (HCT116 and HSC-4) were used, and the cells were treated as in Experimental Example 6 and then observed under a fluorescence microscope. The images observed via fluorescence microscopy are presented in Fig. 14.

(Results)

When the cells were treated with SSZ, BSO, or OXY alone, those with increased intracellular HNE levels were observed at a low frequency. However, when SSZ or BSO was used in combination with OXY, cells with a high level of accumulated intracellular HNE were observed at a high frequency.

Thus, by the combination use of xCT with ALDH inhibitors, cells with a high level of intracellular HNE at a high frequency could be observed. It is considered that the xCT and ALDH inhibitors have a synergistic combined effect on the reduction of the survival of sulfasalazine-resistant cells, as in Example 11.

(Experimental Example 14) *In vivo* combined effects of sulfasalazine (SSZ) and oxyfedrine (OXY)

(Purpose)

In this experimental example, it is shown that the combined effects of SSZ and OXY can be observed *in vivo.*

(Methods)

Tumors were formed in mice using SSZ-resistant oral squamous carcinoma cell line HCT-116 cells in the same manner as in Example 4. The volume (at 7 days and 14 days after transplantation) and weight (at 16 days after transplantation) of the tumors were calculated. The results are plotted as presented in Fig. 15. Images of the tumors were taken at the time of measuring the weight of the tumors.

(Results)

As can be seen from Fig. 15, treatment with each agent alone had little growth-suppressing effect on tumors, and the combined use of SSZ and OXY significantly suppressed tumor growth.

Thus, combined administration of SSZ and OXY can inhibit the growth of tumors derived from SSZ-resistant cells.

(Experimental Example 15) Accumulation of HNE in tumors *in vivo* due to the combined use of sulfasalazine (SSZ) and oxyfedrine (OXY)

(Purpose)

In this experimental example, it is shown that combined treatment with sulfasalazine (SSZ) and oxyfedrine (OXY) markedly increases the HNE level in tumors formed *in vivo* and the frequency of HNE-accumulating cells.

(Methods)

Tumors were formed in mice using SSZ-resistant, oral squamous carcinoma cell line HCT-116 cells in the same manner as in Example 4 and were subjected to the following immunohistochemical analysis at 16 days after transplantation. First, the tumor tissues were fixed with 4% formaldehyde, and paraffin sections were prepared. Then, the sections were permeabilized with 0.2% Triton X100-PBS, washed with PBS, and blocked with 3% BSA-PBS. Subsequently, HNE was stained to brown using the Vectastain Elite Kit (Vector Laboratories), anti-HNE antibody as the primary antibody, and ImmPACT DAB Peroxidase Substrate (Vector Laboratories) as the substrate for the enzyme. The microscopic images are presented in Fig. 16.

(Results)

As can be seen from Fig. 16, cells with high levels of intracellular HNE could be observed at a high frequency when SSZ and OXY were combined, even for *in vivo* tumors. It can be considered that SSZ and OXY have a synergistic combined effect on the suppression of the growth of tumors derived from SSZ-resistant cells, as shown in Example 14.

Thus, the xCT and ALDH inhibitors exerts a synergistic combined effect on the suppression of the growth of tumors derived from sulfasalazine-resistant cells.

(Experimental Example 16) Effect of using ALDH inhibitor when treating tumors with radiation

(Purpose)

In this experimental example, irradiation and ALDH inhibitors is show to have a synergistic effect on the reduction of the cell survival rate and HNW accumulation in sulfasalazine (SSZ)-resistant cells, by irradiating SSZ-resistant cells and simultaneously using an ALDH inhibitor to the cells.

(Methods)

In this experimental example, SSZ-resistant tumor cells (HCT116 and HSC-4) were irradiated with ionizing radiation at a dose of 4, 6, or 10 Gy using an X-ray irradiation system (Hitachi MBR-1520R-4, settings: 150 kV, 20 mA) in the presence of 50 μM oxyfedrine (OXY). In parallel, samples without OXY and ionizing radiation (0 Gy) were processed as a control. After 24 hours, cell survival rate was calculated as in Experimental Example 1. The results are presented in Fig. 17. In addition, intracellular HNE was visualized as in Experimental Example 6. The results are presented in Fig. 18.

As shown in Fig. 17, the cell survival rate was significantly and markedly reduced when cells were irradiated in the presence of OXY, as opposed to irradiation or OXY treatment alone.

In addition, as shown in Fig. 18, high levels of intracellular HNE-accumulating cells were observed at a high frequency by using irradiation and OXY treatment in combination.

Thus, it is considered that the combination of irradiation and OXY treatment results in the intracellular HNE accumulation at a high level, which significantly reduces cell survival rate.

(Experimental Example 17) Correlation between the expression level of Nrf2 and the expression levels of xCT and ALDH

(Purpose)

In this experimental example, it is shown that the expression level of Nrf2 are positively correlated with the expression levels of xCT and ALDH.

(Methods)

Nrf2, xCT, and β-actin expressions were detected in the extracts of SSZ-resistant tumor cells (HCT116, HSC-4, and A549) via Western blotting with primary antibodies against Nrf2, xCT, and β-actin and HRP-conjugated secondary antibody. Chemiluminescence Reagent Plus (Perkin-Elmer Japan) was used for detection. The results are presented in Fig. 19(A).

Next, siRNA against the Nrf2 gene was lipofected into A549 cells to suppress the Nrf2 gene expression. The extracts of the lipofected A549 cells were prepared after 48 hours and the expressions of Nrf2, xCT, ALDH3A1, and β-actin were examined via Western blotting in the same way as above. The following sequences (bases shown in lower case are DNA overhangs) were used as siRNA.

Control: 5'-UUCUCCGAACGUGUCACGUtt-3' (SEQ ID NO. 7) 5'-ACGUGACACGUUCGGAGAAtt-3' (SEQ ID NO. 8)

siNrf2: 5'-UCCUACUGUGAUGUGAAAUtt-3' (SEQ ID NO. 9) 5'-AUUUCACAUCACAGUAGGAgc-3' (SEQ ID NO. 10)

The results are presented in Fig. 19(B).

(Results)

As indicated in Fig. 19(A), Nrf2 was overexpressed in A549 cells along with the overexpression of xCT.

When the expression of the Nrf2 gene was suppressed by siRNA in A549 cells, the expressions of xCT and ALDH3A1 were also suppressed.

Thus, the Nrf2 gene expression level is positively correlated with the xCT and ALDH expression levels. The Nrf2 gene is overexpressed, especially in A549 cells.

(Experimental Example 18) Combined effect of siRNA against the Nrf2 gene, sulfasalazine (SSZ) or buthionine sulfoximine (BSO), and oxyfedrine (OXY)

(Purpose)

Referring to Fig. 11, when the results are compared between the A549 and HCT116 or HSC4, the combined effect of oxyfedrine and sulfasalazine is weaker in the A549 cells. In this experimental example, it is shown that the suppression of the Nrf2 gene enhances the combined effect on the A549 cells.

(Methods)

A549 cells were transfected with siRNA against the Nrf2 gene as in Example 17, or the medium was supplemented with ML385 that is an Nrf2 inhibitor. Cells were cultured for 48 hours in a medium containing 50 μM OXY, and 400 μM SSZ or 100 μM BSO, as in Example 11. Then, the cells were assayed for cell viability. The results are presented in Fig. 20. As a control, the experiments were performed in a medium without any of the above. DMSO, however, was added appropriately to add a constant volume of DMSO in all cases.

(Results)

As shown in Fig. 20, the cell survival rate can be effectively reduced in A549 cells by administering siRNA against the Nrf2 gene or ML385, an Nrf2 inhibitor, in addition to SSZ and OXY.

Thus, the Nrf2 gene expression can be an indicator of whether co-administration of SSZ and OXY is effective in suppressing tumor growth. Then, the effect of SSZ and OXY can be enhanced by administering siRNA against the Nrf2 gene or ML385 in addition to SSZ and OXY. This strategy is particularly effective for tumors overexpressing the Nrf2 gene.

(Experimental Example 19) Effect of using ALDH inhibitors in tumor radiotherapy

(Purpose)

In this experimental example, it is shown that irradiation of tumor cells transplanted in nude mice in combination with the use of an ALDH inhibitor has a synergistic effect on the reduction of cell viability and HNE accumulation in sulfasalazine (SSZ)-resistant cells, by irradiating SSZ-resistant cells and simultaneously using an ALDH inhibitor to the cells.

(Methods)

1.5 × $10^6$ cells of sulfasalazine-resistant, oral squamous carcinoma cell line HSC-2 were subcutaneously transplanted in nude mice (five animals). The mice were injected intraperitoneally with oxyfedrine (OXY) (20 mg/kg) for 3 consecutive days (Days 1-3) immediately after transplantation. On Day4, oxyfedrine (30 mg/kg) was intraperitoneally administered, and X-rays (4, 6, or 10 Gy) were irradiated to the nude mice 2 hours later. Then, after intraperitoneal administration of oxyfedrine (20 mg/kg) for 3 days (Days 5-7), tumors were collected on Day 7 and weighed. The results are shown in a bar graph in Fig. 21. The groups of nude mice on which transplantation was performed in the same way, to which oxyfedrine was administered in the same way without X-irradiation, and to which no oxyfedrine was administered with X-irradiation in the same way, were used as controls. For statistical treatment, Student's *t*-test was performed between the oxyfedrine and non-treated groups for each X-ray dose, and *p* value of less than 0.05 was considered significant.

(Results)

As shown in Fig. 21, the tumor weight gain was suppressed as the X-ray dose was increased. In the groups without X-irradiation and the 4 Gy X-irradiation group, tumor weight gain tended to be suppressed in the oxyfedrine group compared with the non-treated group, but in the 6 Gy and 10 Gy X-irradiation groups, tumor weight gain was significantly suppressed in the oxyfedrine group compared with the non-treated group.

Thus, irradiation and oxyfedrine administration have a synergistic effect on the suppression of tumor weight gain *in vivo.*

Industrial Applicability

The present invention made it possible to provide novel anti-tumor agents and combination products.

## Claims

1. An anti-tumor agent comprising, as an active ingredient, a glutathione level reducer or a glutathione S-transferase inhibitor, the anti-tumor agent being adapted to be administered simultaneously with an effective amount of an aldehyde dehydrogenase inhibitor, or a compound (II) below or a pharmacologically acceptable salt thereof:

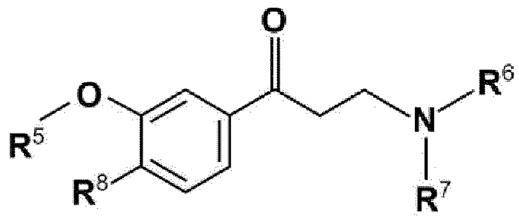

(I I)

where $R^5$ is a linear or branched C1-6 alkyl group, $R^6$ is hydrogen or halogen, $R^7$ is a linear or branched C1-6 alkyl group optionally substituted with a substituent, the substituent being hydroxy or phenyl, and $R^8$ is hydrogen or halogen.

2. An anti-tumor agent comprising, as an active ingredient, an aldehyde dehydrogenase inhibitor, or a compound (II) below or a pharmacologically acceptable salt thereof, the anti-tumor agent being adapted to be administered simultaneously with an effective amount of a glutathione level reducer or a glutathione S-transferase inhibitor:

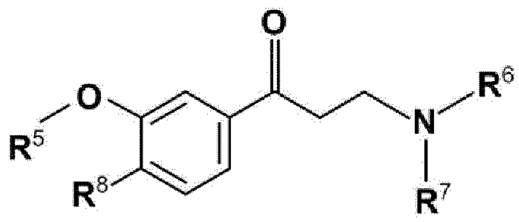

(I I)

where $R^5$ is a linear or branched C1-6 alkyl group, $R^2$ and $R^3$ are each independently selected from linear and branched C1-6 alkyl groups, or $R^2$ and $R^3$ together with nitrogen as a heteroatom to which they are attached form a 4-, 5-, 6-, or 7-membered azacycloalkyl group, and $R^4$ is hydrogen or halogen.

3. The anti-tumor agent according to Claim 1 or 2, wherein the glutathione level reducer inhibits activity of any one or more of xCT, thioredoxin-1 (TRX-1), glutamate-cysteine ligase (GCL) (EC 6.3.2.2) (also called γ-glutamylcysteine synthetase), and glutathione synthetase (EC 6.3.2.3).

4. The anti-tumor agent according to Claim 3, wherein the glutathione level reducer is an xCT inhibitor or a GCL inhibitor.

5. The anti-tumor agent according to Claim 4, wherein the glutathione level reducer is sulfasalazine or L-buthionine-sulfoximine.

6. The anti-tumor agent according to any one of Claims 1 to 5, wherein the compound represented by the formula (II) is oxyfedrine.

7. A combination drug comprising, as active ingredients, an aldehyde dehydrogenase inhibitor, or a compound (II) below or a pharmacologically acceptable salt thereof; and a glutathione level reducer or a glutathione S-transferase

inhibitor:

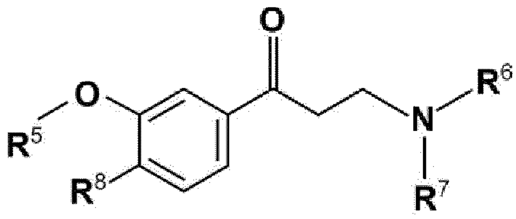

(I I)

where $R^5$ is a linear or branched C1-6 alkyl group, $R^6$ is hydrogen or halogen, $R^7$ is a linear or branched C1-6 alkyl group optionally substituted with a substituent, the substituent being hydroxy or phenyl, and $R^8$ is hydrogen or halogen.

**8.** The combination drug according to Claim 7, wherein the glutathione level reducer inhibits activity of any one or more of xCT, thioredoxin-1 (TRX-1), glutamate-cysteine ligase (GCL) (EC 6.3.2.2) (also called γ-glutamylcysteine synthetase), and glutathione synthetase (EC 6.3.2.3).

**9.** The combination drug according to Claim 8, wherein the glutathione level reducer is an xCT inhibitor or a GCL inhibitor.

**10.** The combination drug according to Claim 9, wherein the glutathione level reducer is sulfasalazine or L-buthionine-sulfoximine.

**11.** The combination drug according to Claim 10, wherein the compound represented by the formula (II) is oxyfedrine.

**12.** An anti-tumor agent comprising the combination drug according to any one of Claims 7 to 11.

**13.** The anti-tumor agent according to any one of Claims 1 to 6 and 12, wherein the agent is against a tumor comprising tumor cells resistant to a glutathione level reducer or a glutathione S-transferase inhibitor.

**14.** The anti-tumor agent according to Claim 13, wherein the tumor cells have high expression of aldehyde dehydrogenase.

**15.** The anti-tumor agent according to Claim 13 or 14, wherein the tumor further comprises tumor cells expressing CD44v.

**16.** An assay method comprising the steps of:

simultaneously administering an aldehyde dehydrogenase inhibitor, or a compound (III) or a pharmacologically acceptable salt thereof, and a glutathione level reducer or a glutathione S-transferase inhibitor, to a tumor cell *in vitro;* and
measuring a growth rate or a cell survival rate of the tumor cell:

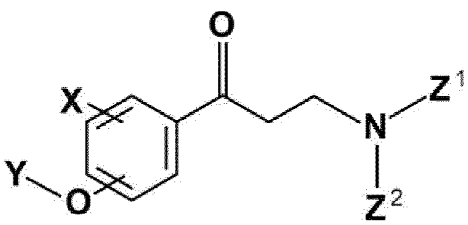

(I I I)

where X is hydrogen, halogen, $-NH_2$, or -CN, Y is a linear or branched C1-6 alkyl group, and $Z^1$ and $Z^2$ are hydrogen or halogen and a linear or branched C1-6 alkyl group optionally substituted with a substituent, respectively, the substituent being hydroxy or phenyl, or $Z^1$ and $Z^2$ together with nitrogen as a heteroatom to which they are attached form a 4-, 5-, 6-, or 7-membered azacycloalkyl group.

**17.** A method of identifying an agent that induces a combined effect with a glutathione level reducer or a glutathione S-transferase inhibitor, comprising the steps of:

simultaneously administering a given glutathione level reducer or a given glutathione S-transferase inhibitor and each of a plurality of aldehyde dehydrogenase inhibitors, or compounds (III) or pharmacologically acceptable salts thereof, to tumor cells *in vitro;* and
measuring a growth rate or a cell survival rate of the tumor cells:

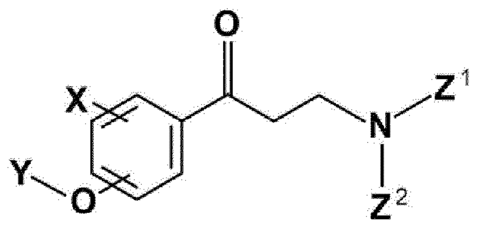

(III)

where X is hydrogen, halogen, $-NH_2$, or -CN, Y is a linear or branched C1-6 alkyl group, and $Z^1$ and $Z^2$ are hydrogen or halogen and a linear or branched C1-6 alkyl group optionally substituted with a substituent, respectively, the substituent being hydroxy or phenyl, or $Z^1$ and $Z^2$ together with nitrogen as a heteroatom to which they are attached form a 4-, 5-, 6-, or 7-membered azacycloalkyl group.

**18.** A method for identifying a glutathione level reducer or a glutathione-S-transferase inhibitor that induces a combined effect with a compound (III) or a pharmacologically acceptable salt thereof, the compound (III) being an anti-tumor agent, the method comprising the steps of:

simultaneously administering the compound (III) and a plurality of glutathione level reducers or glutathione S-transferase inhibitors, to tumor cells *in vitro;* and
measuring a growth rate or a cell survival rate of the tumor cells:

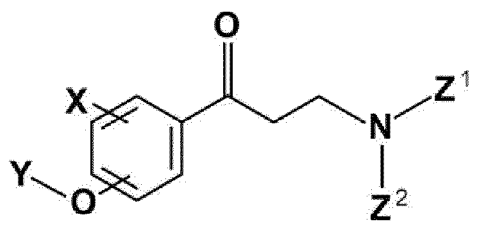

(III)

where X is hydrogen, halogen, $-NH_2$, or -CN, Y is a linear or branched C1-6 alkyl group, and $Z^1$ and $Z^2$ are hydrogen or halogen and a linear or branched C1-6 alkyl group optionally substituted with a substituent, respectively, the substituent being hydroxy or phenyl, or $Z^1$ and $Z^2$ together with nitrogen as a heteroatom to which they are attached form a 4-, 5-, 6-, or 7-membered azacycloalkyl group.

**19.** The method according to Claim 17 or 18, wherein the compound (III) is a compound (II):

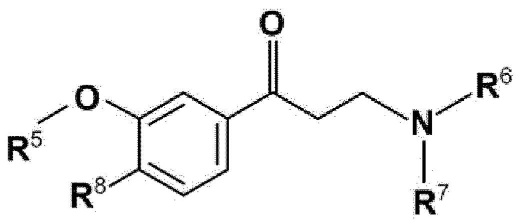

(II)

where $R^5$ is a linear or branched C1-6 alkyl group, $R^2$ and $R^3$ are each independently selected from linear and branched C1-6 alkyl groups, or $R^2$ and $R^3$ together with nitrogen as a heteroatom to which they are attached form a 4-, 5-, 6-, or 7-membered azacycloalkyl group, and $R^4$ is hydrogen or halogen.

**20.** A method for identifying a kind of tumor cells on which a compound (III) or a pharmacologically acceptable salt thereof and a glutathione level reducer or a glutathione S-transferase inhibitor have a combined effect, the compound (III) being an anti-tumor agent, the method comprising the steps of:

simultaneously administering a given combination of the compound (III) or a pharmacologically acceptable salt thereof and a glutathione level reducer or a glutathione S-transferase inhibitor, to different kinds of tumor cells *in vitro;* and
measuring a growth rate or a cell survival rate of the different kinds of tumor cells.

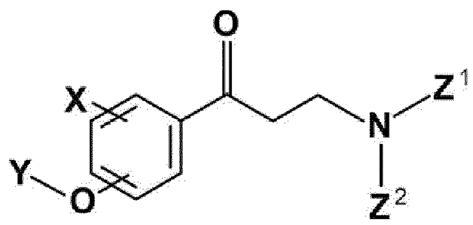

(III)

where X is hydrogen, halogen, $-NH_2$, or -CN, Y is a linear or branched C1-6 alkyl group, and $Z^1$ and $Z^2$ are hydrogen or halogen and a linear or branched C1-6 alkyl group optionally substituted with a substituent, respectively, the substituent being hydroxy or phenyl, or $Z^1$ and $Z^2$ together with nitrogen as a heteroatom to which they are attached form a 4-, 5-, 6-, or 7-membered azacycloalkyl group.

**21.** The method according to Claim 20, wherein the compound (III) is a compound (II):

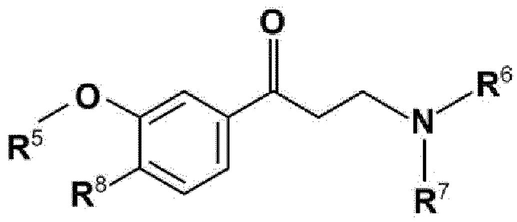

(II)

where $R^5$ is a linear or branched C1-6 alkyl group, $R^2$ and $R^3$ are each independently selected from linear and branched C1-6 alkyl groups, or $R^2$ and $R^3$ together with nitrogen as a heteroatom to which they are attached form a 4-, 5-, 6-, or 7-membered azacycloalkyl group, and $R^4$ is hydrogen or halogen.

**22.** The assay method according to Claim 16, wherein the tumor cells are resistant to a glutathione level reducer or a glutathione S-transferase inhibitor.

**23.** The method according to any one of Claims 17 to 21, wherein the tumor cells are resistant to a glutathione level reducer or a glutathione S-transferase inhibitor.

**24.** An anti-tumor agent used in radiotherapy, comprising, as an active ingredient, an aldehyde dehydrogenase inhibitor, or a compound (II) below or a pharmacologically acceptable salt thereof:

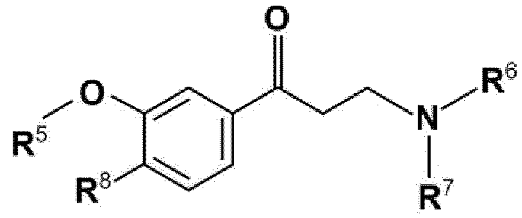

(I I)

where $R^5$ is a linear or branched C1-6 alkyl group, $R^2$ and $R^3$ are each independently selected from linear and branched C1-6 alkyl groups, or $R^2$ and $R^3$ together with nitrogen as a heteroatom to which they are attached form a 4-, 5-, 6-, or 7-membered azacycloalkyl group, and $R^4$ is hydrogen or halogen.

**25.** The anti-tumor agent according to Claim 24, wherein the compound represented by the formula (II) is oxyfedrine.

**26.** The anti-tumor agent according to Claim 24 or 25, wherein the agent is against a tumor comprising tumor cells resistant to a glutathione level reducer or a glutathione S-transferase inhibitor.

**27.** The anti-tumor agent according to Claim 26, wherein the tumor cells have high expression of aldehyde dehydrogenase.

**28.** The anti-tumor agent according to Claim 26 or 27, wherein the tumor further comprises tumor cells expressing CD44v.

**29.** An assay method for an anti-tumor effect comprising the steps of:

exposing tumor cells *in vitro* to a radiation in the presence of an aldehyde dehydrogenase inhibitor, or a compound (III) or a pharmacologically acceptable salt thereof; and
measuring a growth rate or a cell survival rate of the tumor cells:

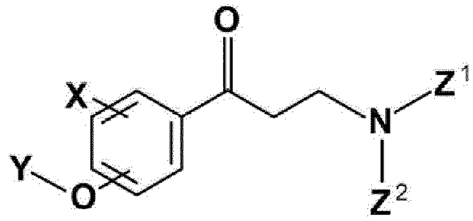

(I I I)

where X is hydrogen, halogen, $-NH_2$, or -CN, Y is a linear or branched C1-6 alkyl group, and $Z^1$ and $Z^2$ are hydrogen or halogen and a linear or branched C1-6 alkyl group optionally substituted with a substituent, respectively, the substituent being hydroxy or phenyl, or $Z^1$ and $Z^2$ together with nitrogen as a heteroatom to which they are attached form a 4-, 5-, 6-, or 7-membered azacycloalkyl group.

**30.** The assay method according to Claim 29, wherein the tumor cells are resistant to a glutathione level reducer or a glutathione S-transferase inhibitor.

**31.** A method for identifying an agent having a synergistic effect with irradiation in tumor cells *in vitro*, the method comprising the steps of:

exposing tumor cells *in vitro* to a radiation in the presence of an aldehyde dehydrogenase inhibitor, or a compound (III) or a pharmacologically acceptable salt thereof; and
measuring a growth rate or a cell survival rate of the tumor cells:

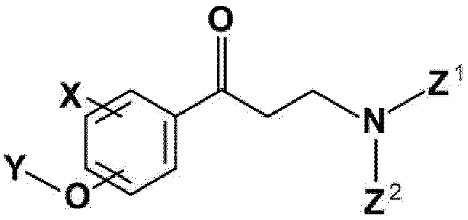

(III)

where X is hydrogen, halogen, $-NH_2$, or -CN, Y is a linear or branched C1-6 alkyl group, and $Z^1$ and $Z^2$ are hydrogen or halogen and a linear or branched C1-6 alkyl group optionally substituted with a substituent, respectively, the substituent being hydroxy or phenyl, or $Z^1$ and $Z^2$ together with nitrogen as a heteroatom to which they are attached form a 4-, 5-, 6-, or 7-membered azacycloalkyl group.

**32.** The method according to Claim 31, wherein the compound (III) is a compound (II).

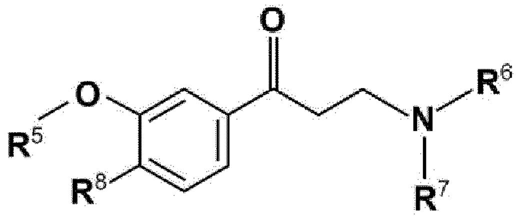

(II)

where $R^5$ is a linear or branched C1-6 alkyl group, $R^2$ and $R^3$ are each independently selected from linear and branched C1-6 alkyl groups, or $R^2$ and $R^3$ together with nitrogen as a heteroatom to which they are attached form a 4-, 5-, 6-, or 7-membered azacycloalkyl group, and $R^4$ is hydrogen or halogen.

**33.** The method according to Claim 31 or 32, wherein the tumor cells are resistant to a glutathione level reducer or a glutathione S-transferase inhibitor.

**34.** An enhancer potentiating an anti-tumor action caused by co-administration with an aldehyde dehydrogenase inhibitor, or a compound (II) below or a pharmacologically acceptable salt thereof, and a glutathione level reducer or a glutathione S-transferase inhibitor, the enhancer comprising a suppressor of an xCT expression-enhancing action of Nrf2:

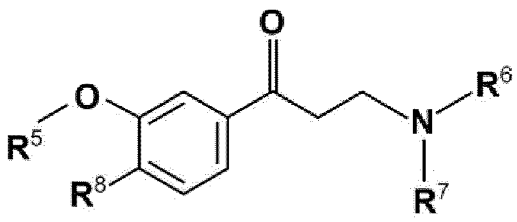

(II)

where $R^5$ is a linear or branched C1-6 alkyl group, $R^6$ is hydrogen or halogen, $R^7$ is a linear or branched C1-6 alkyl group optionally substituted with a substituent, the substituent being hydroxy or phenyl, and $R^8$ is hydrogen or halogen.

**35.** The enhancer according to Claim 34, wherein the suppressor is an Nrf2-gene expression suppressing substance or an Nrf2 inhibitor.

**36.** The enhancer according to Claim 35, wherein the Nrf2-gene expression suppressing substance is an antisense NA, miNA, or siNA against an Nrf2 gene.

**37.** The enhancer according to Claim 35, wherein the Nrf2 inhibitor is ML385 or an anti-Nrf2 antibody.

**38.** The enhancer according to any one of Claims 34 to 37, wherein the anti-tumor effect is on a tumor overexpressing an Nrf2 gene.

**39.** The enhancer according to any one of Claims 34 to 38, wherein the glutathione level reducer is sulfasalazine.

**40.** The anti-tumor agent according to any one of Claims 34 to 39, wherein the compound represented by the formula (II) is oxyfedrine.

**41.** A companion diagnostic drug for predicting an anti-tumor effect upon co-administration of an aldehyde dehydrogenase inhibitor, or a compound (II) below or a pharmacologically acceptable salt thereof, and a glutathione level reducer or a glutathione S-transferase inhibitor, the companion diagnostic drug comprising a detection reagent for detecting Nrf2 gene expression:

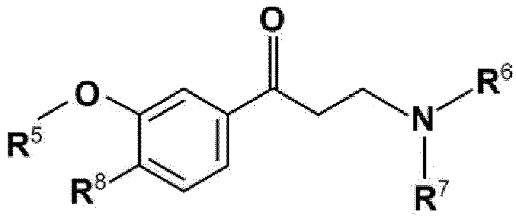

(I I)

where $R^5$ is a linear or branched C1-6 alkyl group, $R^6$ is hydrogen or halogen, $R^7$ is a linear or branched C1-6 alkyl group optionally substituted with a substituent, the substituent being hydroxy or phenyl, and $R^8$ is hydrogen or halogen.

**42.** The companion diagnostic drug according to Claim 41, wherein the detection reagent is an antibody, a probe for detecting gene expression, or a primer for gene amplification.

**43.** The companion diagnostic drug for anti-tumor effect according to Claim 41 or 42, wherein the glutathione level reducer is sulfasalazine.

**44.** The companion diagnostic drug according to any one of Claims 41 to 43, wherein the compound represented by the formula (II) is oxyfedrine.

**45.** A companion diagnostic drug for predicting an anti-tumor effect upon co-administration of an aldehyde dehydrogenase inhibitor, or a compound (II) below or a pharmacologically acceptable salt thereof, and a glutathione level reducer or a glutathione S-transferase inhibitor, the companion diagnostic drug comprising a detection reagent for detecting a mutation of Keap1 or Nrf2 gene:

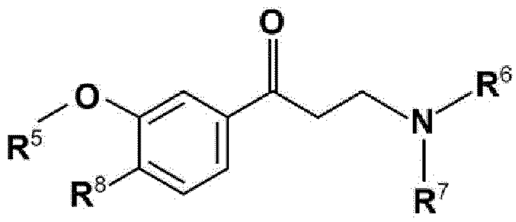

(I I)

where $R^5$ is a linear or branched C1-6 alkyl group, $R^6$ is hydrogen or halogen, $R^7$ is a linear or branched C1-6 alkyl group optionally substituted with a substituent, the substituent being hydroxy or phenyl, and $R^8$ is hydrogen or halogen.

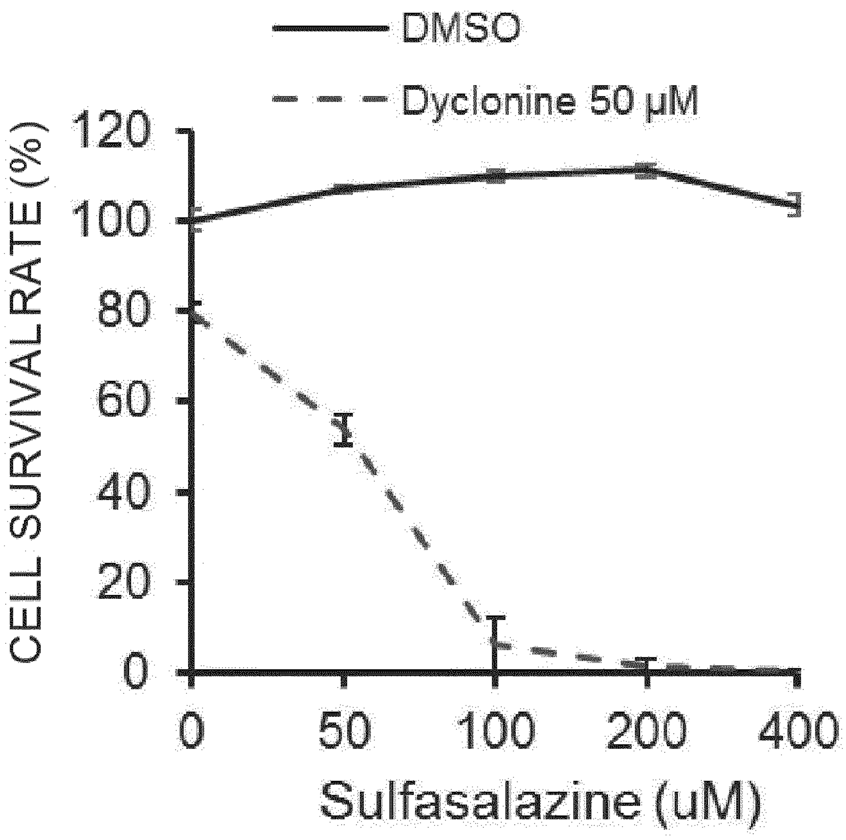
DMSO
Dyclonine 50 μM
CELL SURVIVAL RATE (%)
120
100
80
60
40
20
0
0
50
100
200
400
Sulfasalazine (uM)

FIG. 1

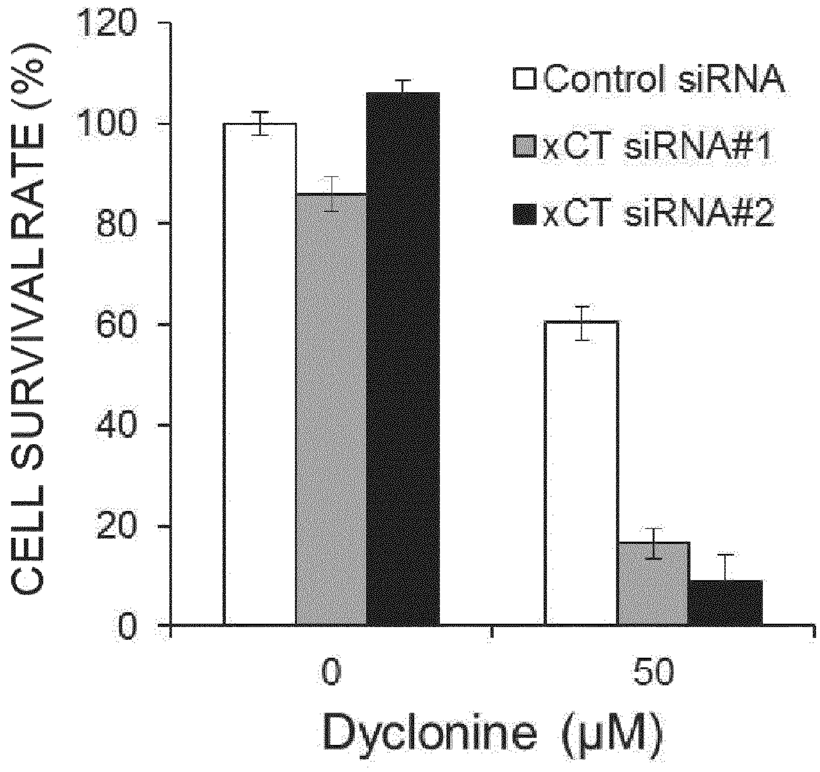
120
100
80
60
40
20
0
CELL SURVIVAL RATE (%)
Control siRNA
xCT siRNA#1
xCT siRNA#2
0
50
Dyclonine (μM)

FIG. 2

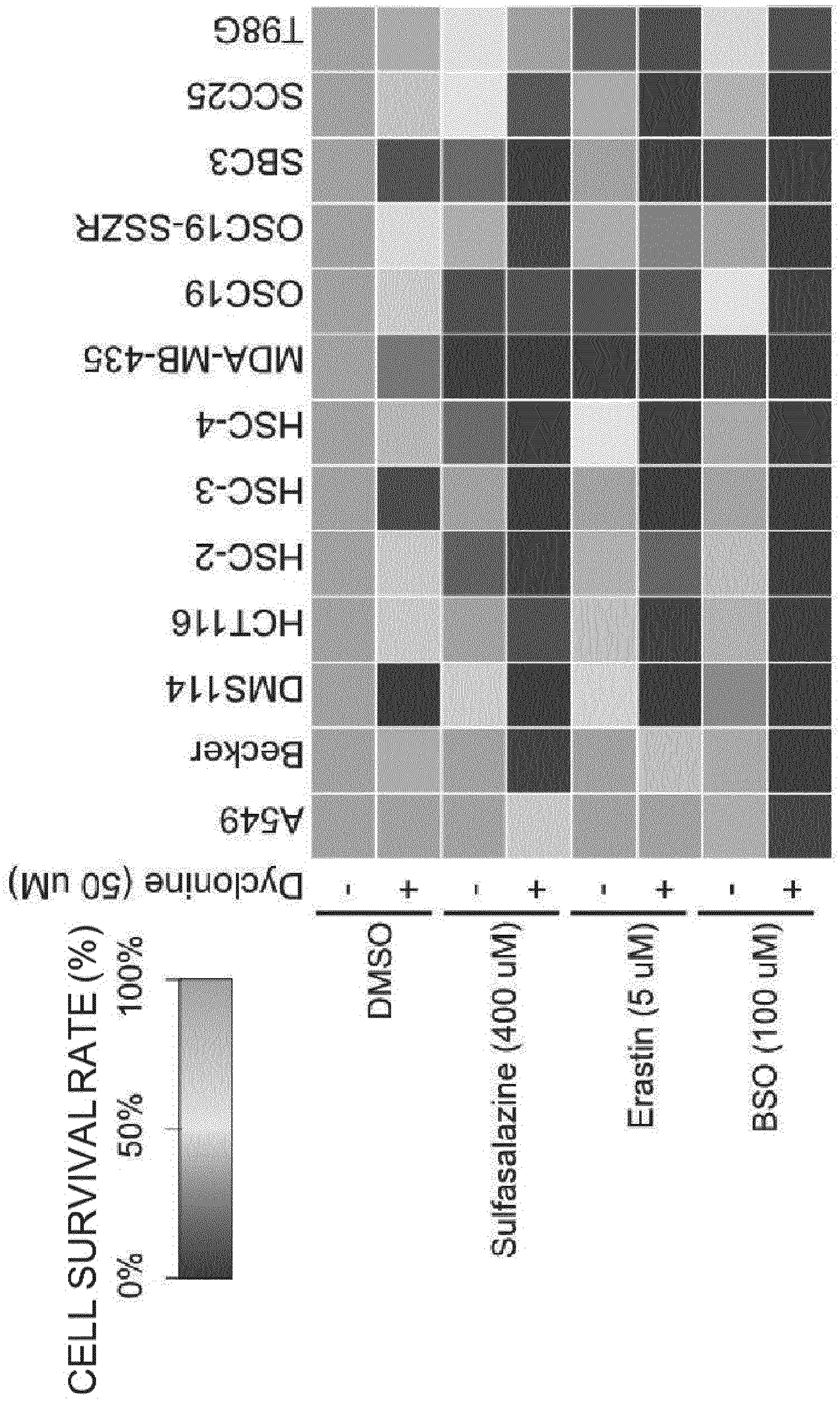
T98G
SCC25
SBC3
OSC19-SSZR
OSC19
MDA-MB-435
HSC-4
HSC-3
HSC-2
HCT116
DMS114
Becker
A549
Dyclonine (50 uM)
- + - + - + - +
DMSO
Sulfasalazine (400 uM)
Erastin (5 uM)
BSO (100 uM)
CELL SURVIVAL RATE (%)
0%
50%
100%

FIG. 3

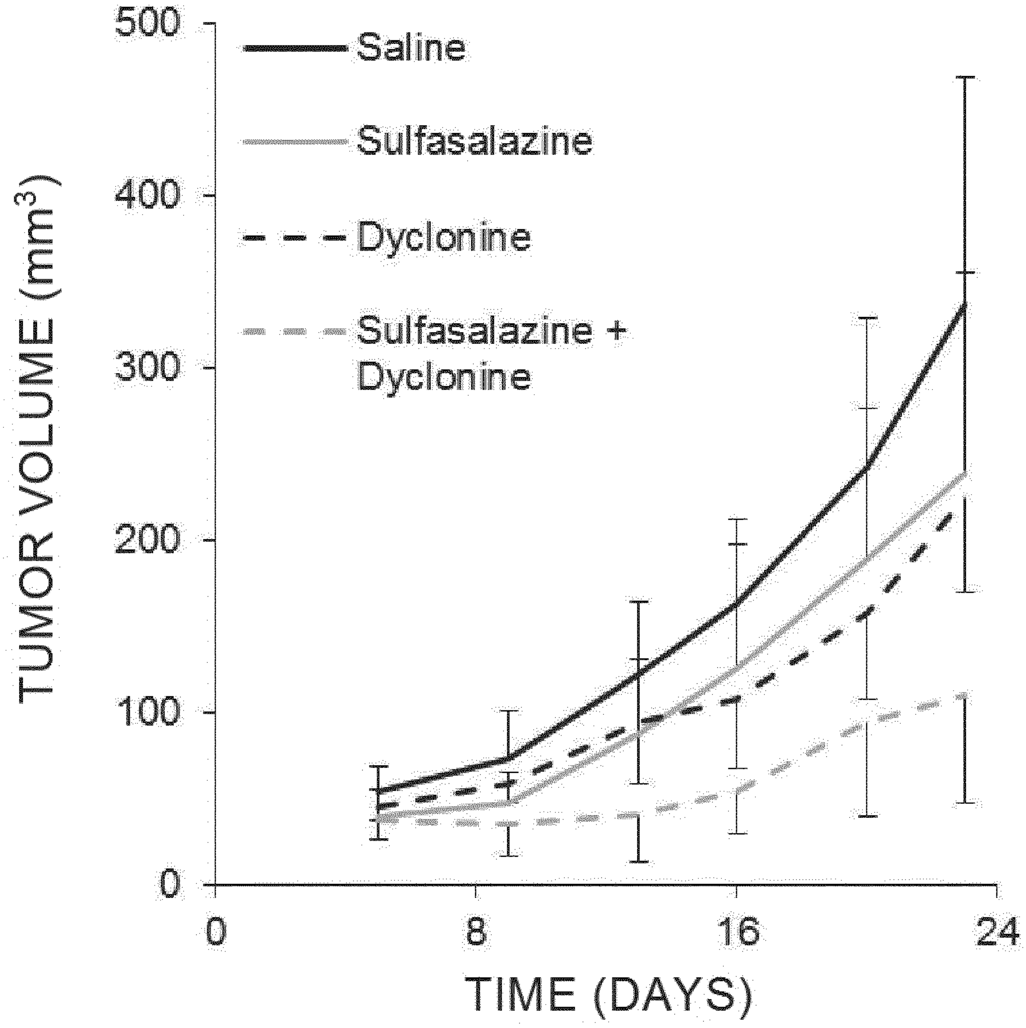

500
400
300
200
100
0
TUMOR VOLUME (mm3)
0
8
16
24
TIME (DAYS)
Saline
Sulfasalazine
Dyclonine
Sulfasalazine +
Dyclonine


FIG. 4

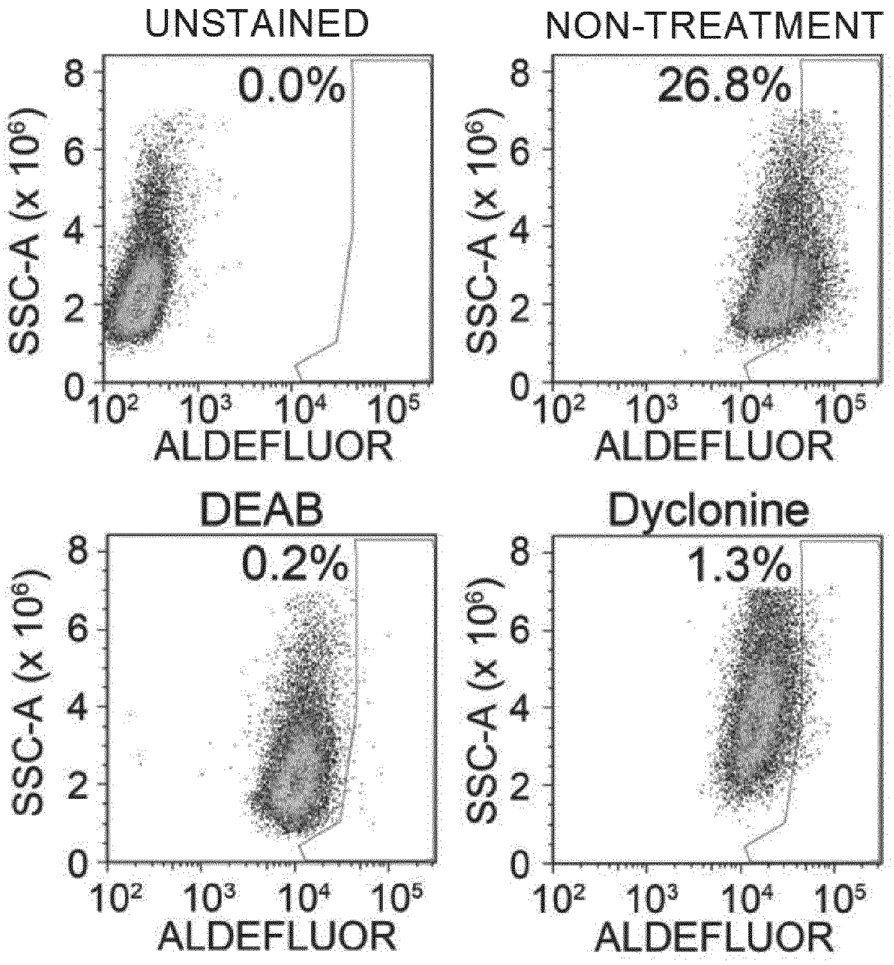
UNSTAINED
0.0%
NON-TREATMENT
26.8%
DEAB
0.2%
Dyclonine
1.3%
SSC-A (x 10^6)
ALDEFLUOR
0
2
4
6
8
10^2
10^3
10^4
10^5

FIG. 5

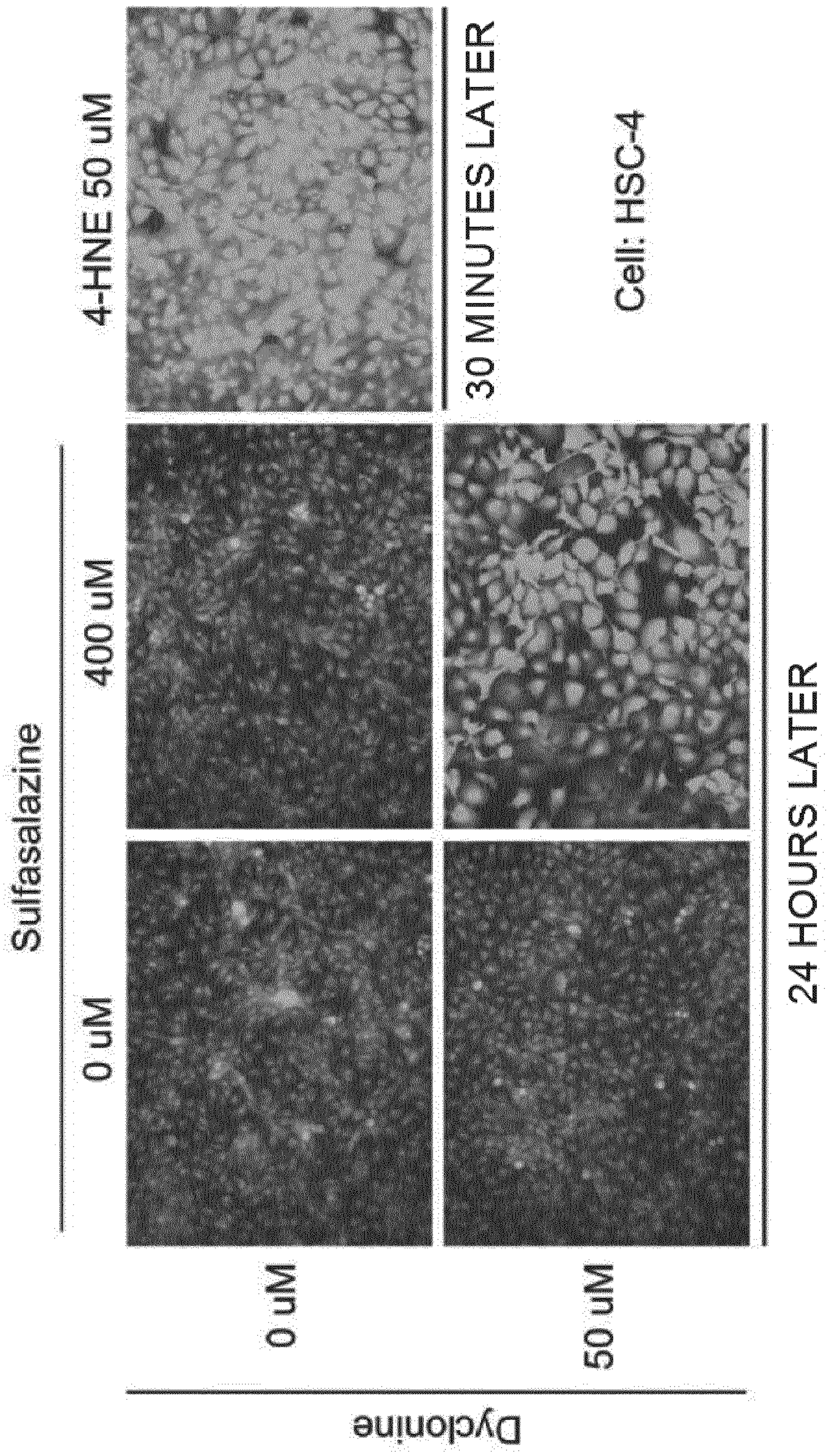
Sulfasalazine
0 uM
400 uM
4-HNE 50 uM
30 MINUTES LATER
Cell: HSC-4
24 HOURS LATER
Dyclonine
0 uM
50 uM

FIG. 6

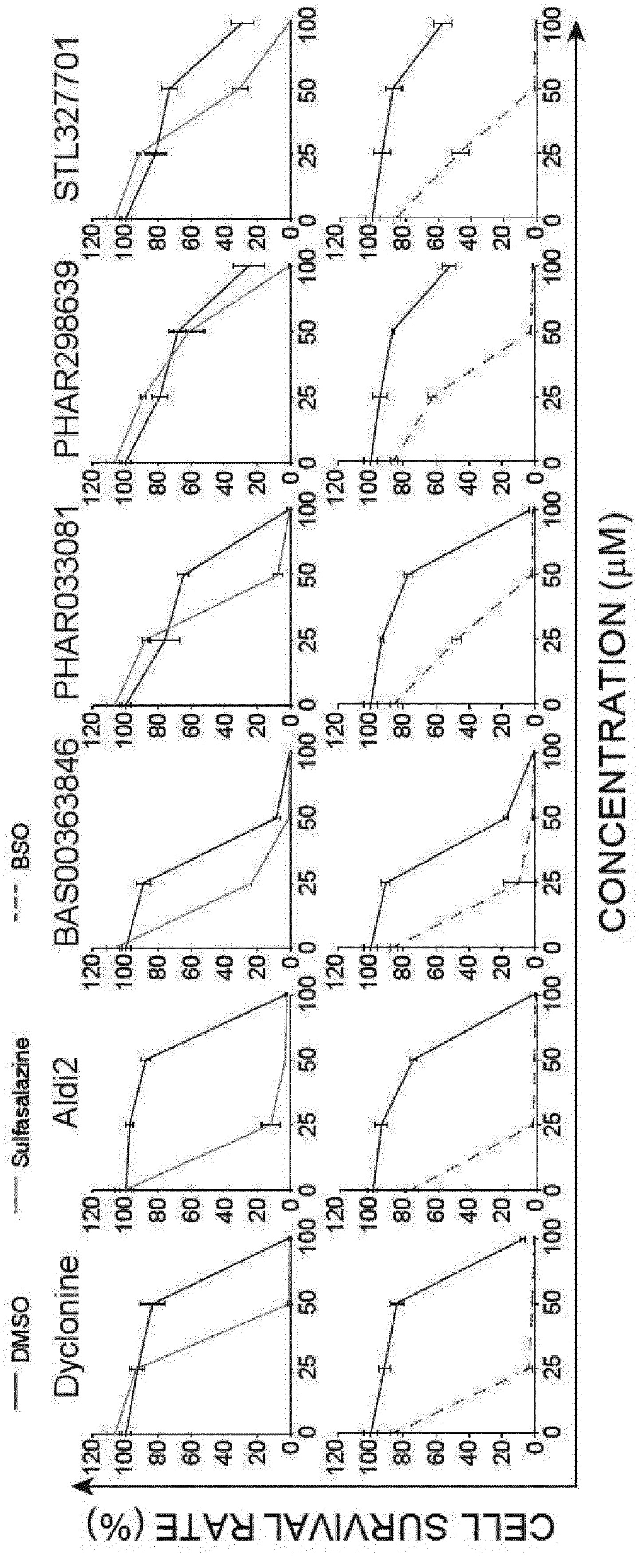
DMSO
Sulfasalazine
BSO
Dyclonine
Aldi2
BAS00363846
PHAR033081
PHAR298639
STL327701
CELL SURVIVAL RATE (%)
CONCENTRATION (μM)

FIG. 7A

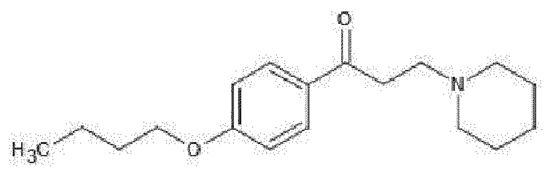
Dyclonine

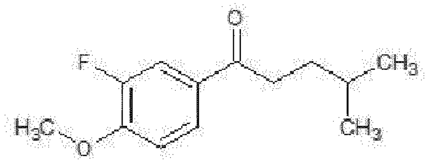
Aldi-2

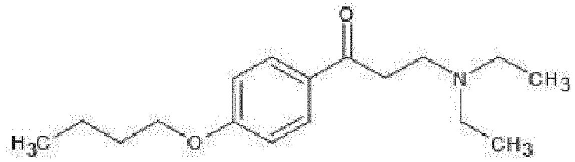
BAS00363846

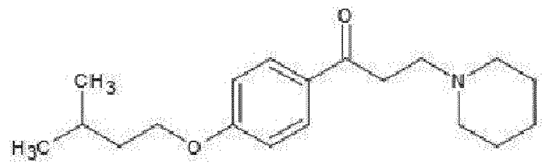
PHAR033081

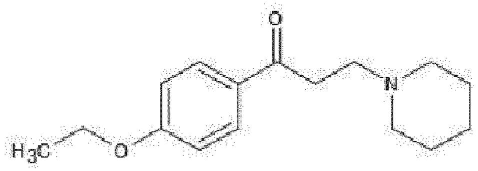
PHAR298639

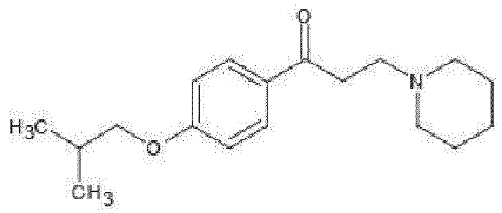
STL327701

FIG. 7B

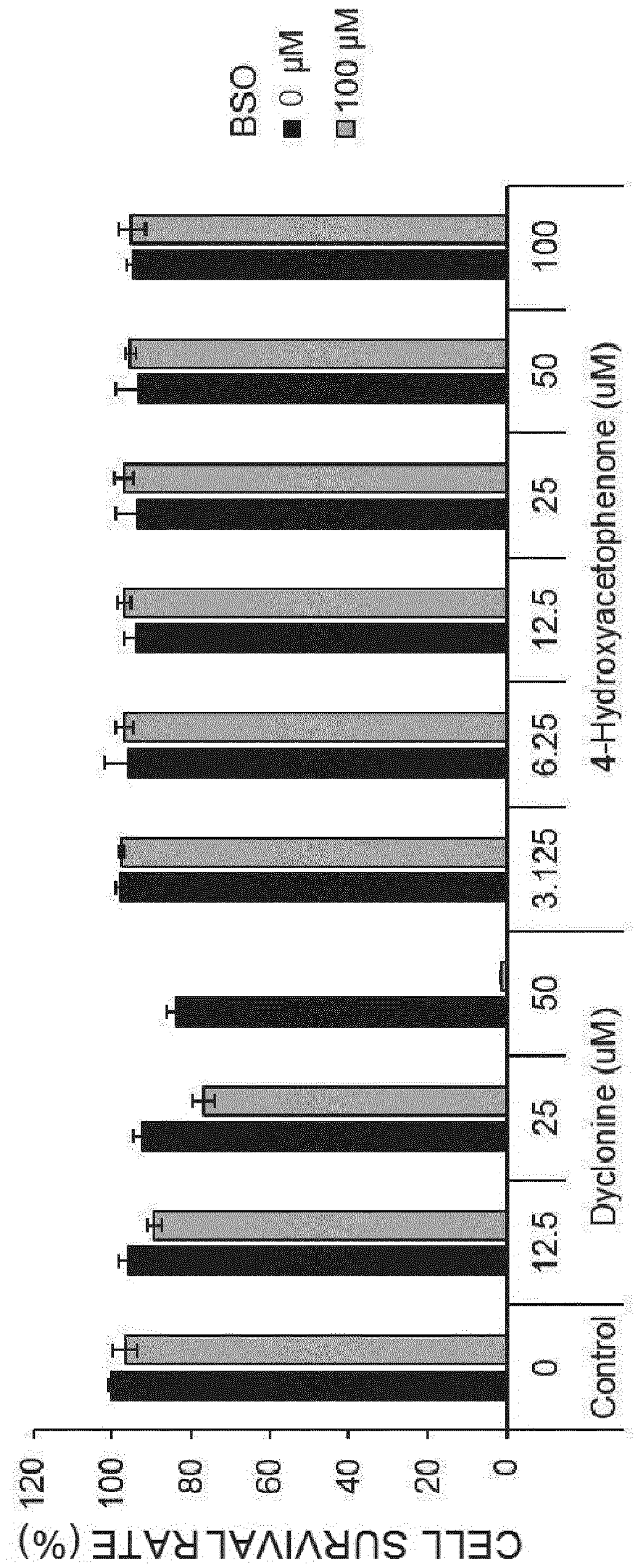


FIG. 8A

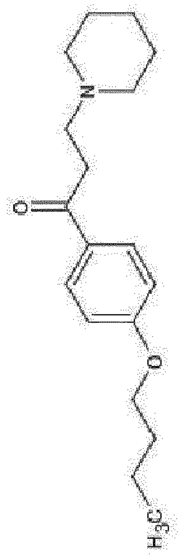
Dyclonine

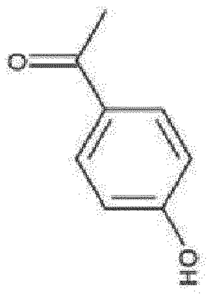
4'-Hydroxyacetophenone

FIG. 8B

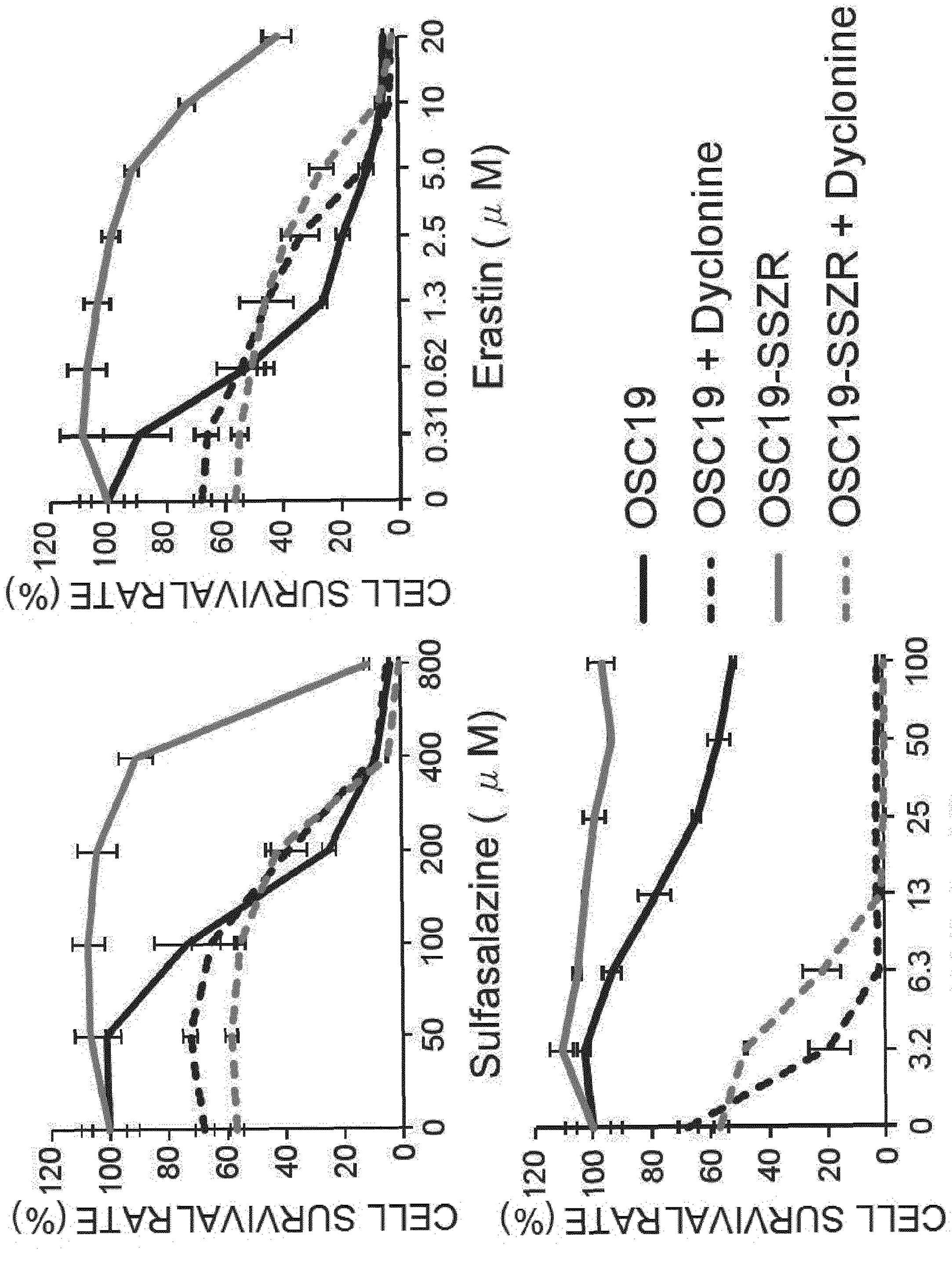
CELL SURVIVALRATE (%)
Sulfasalazine ( μ M)
CELL SURVIVALRATE (%)
Erastin ( μ M)
CELL SURVIVALRATE (%)
BSO ( μ M)
OSC19
OSC19 + Dyclonine
OSC19-SSZR
OSC19-SSZR + Dyclonine

FIG. 9

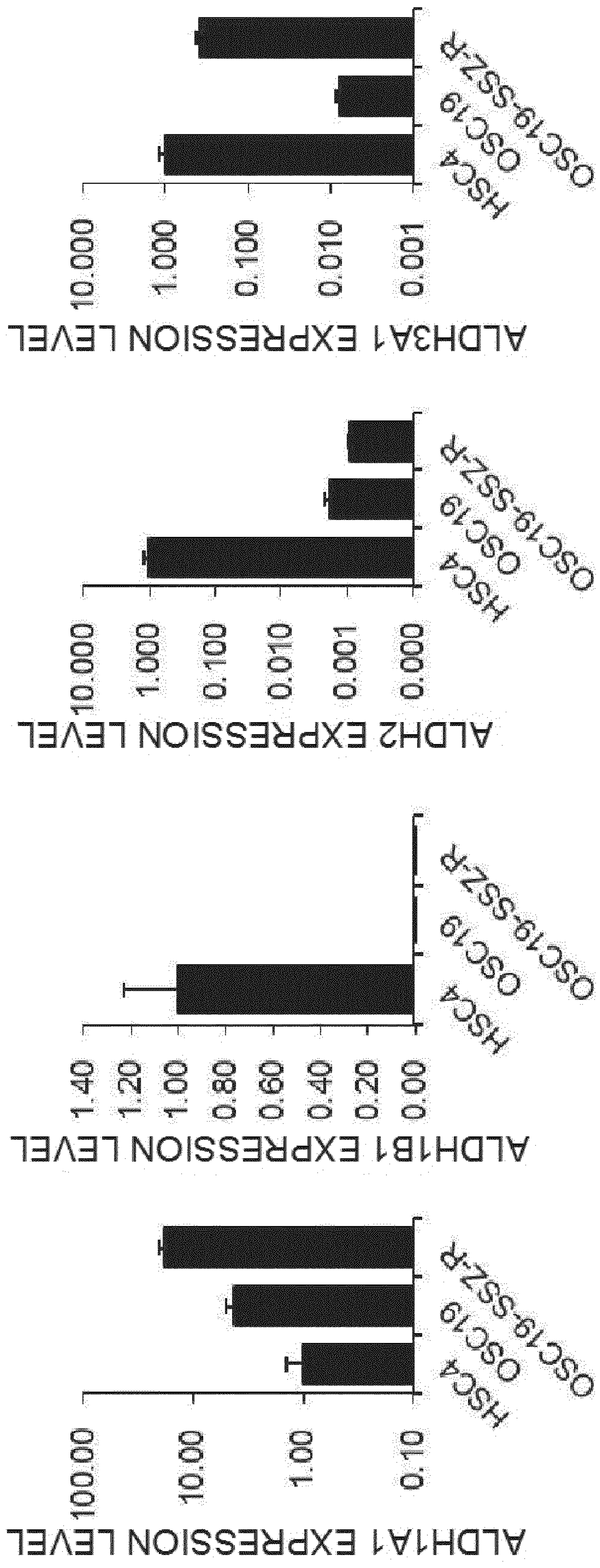
ALDH1A1 EXPRESSION LEVEL
100.00
10.00
1.00
0.10
HSC4
OSC19
OSC19-SSZ-R
ALDH1B1 EXPRESSION LEVEL
1.40
1.20
1.00
0.80
0.60
0.40
0.20
0.00
HSC4
OSC19
OSC19-SSZ-R
ALDH2 EXPRESSION LEVEL
10.000
1.000
0.100
0.010
0.001
0.000
HSC4
OSC19
OSC19-SSZ-R
ALDH3A1 EXPRESSION LEVEL
10.000
1.000
0.100
0.010
0.001
HSC4
OSC19
OSC19-SSZ-R

FIG. 10

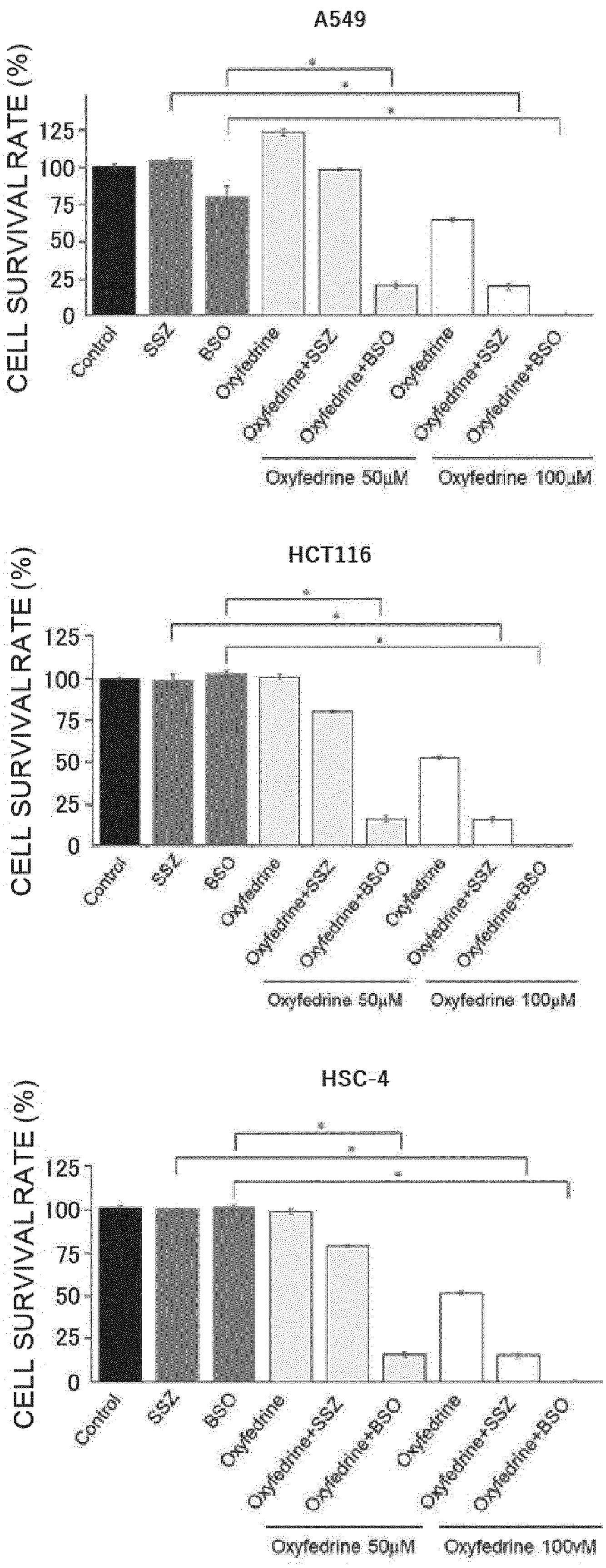
A549
CELL SURVIVAL RATE (%)
125
100
75
50
25
0
*
*
*
Control
SSZ
BSO
Oxyfedrine
Oxyfedrine+SSZ
Oxyfedrine+BSO
Oxyfedrine
Oxyfedrine+SSZ
Oxyfedrine+BSO
Oxyfedrine 50µM
Oxyfedrine 100µM
HCT116
CELL SURVIVAL RATE (%)
125
100
75
50
25
0
*
*
*
Control
SSZ
BSO
Oxyfedrine
Oxyfedrine+SSZ
Oxyfedrine+BSO
Oxyfedrine
Oxyfedrine+SSZ
Oxyfedrine+BSO
Oxyfedrine 50µM
Oxyfedrine 100µM
HSC-4
CELL SURVIVAL RATE (%)
125
100
75
50
25
0
*
*
*
Control
SSZ
BSO
Oxyfedrine
Oxyfedrine+SSZ
Oxyfedrine+BSO
Oxyfedrine
Oxyfedrine+SSZ
Oxyfedrine+BSO
Oxyfedrine 50µM
Oxyfedrine 100vM

FIG. 11

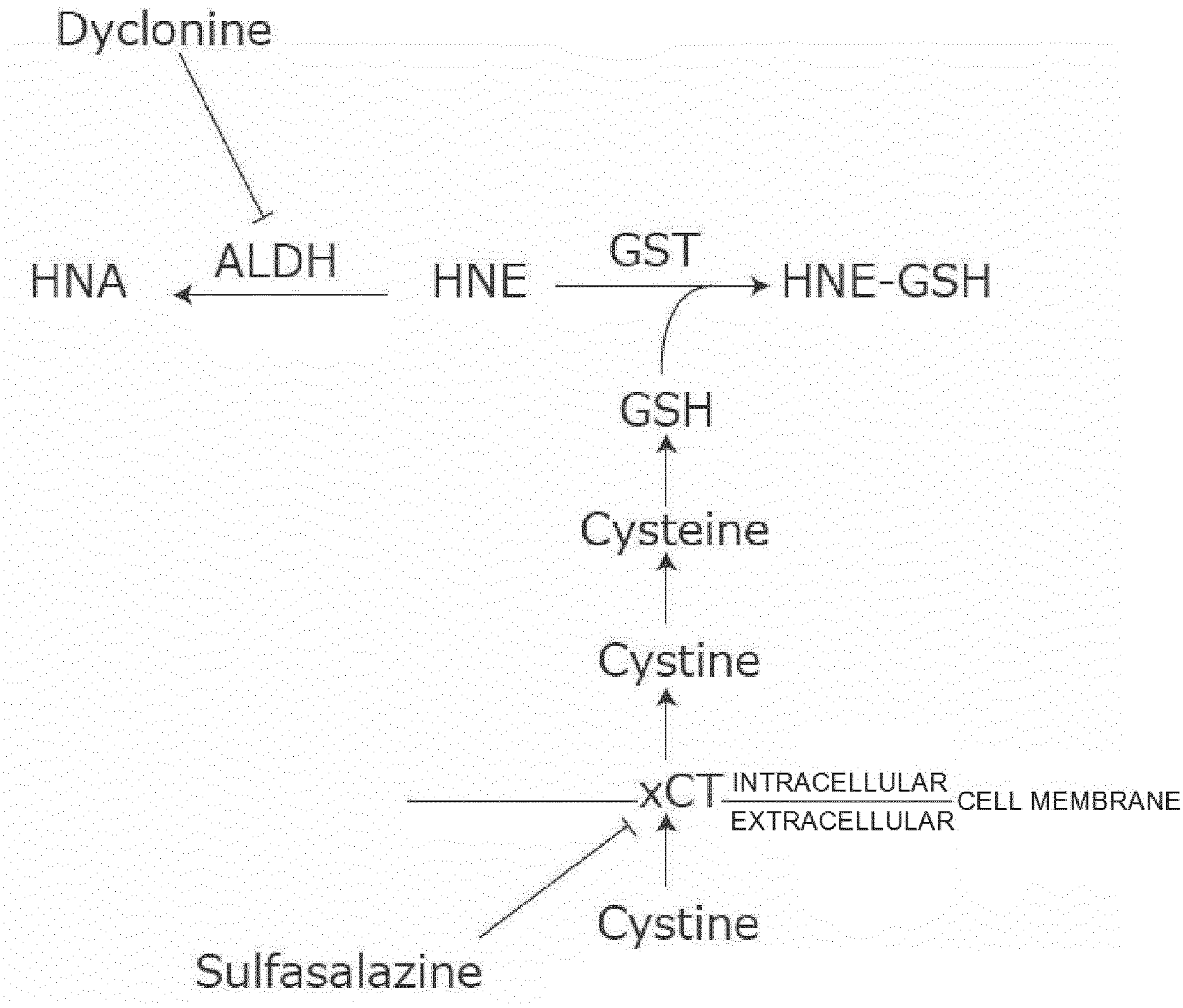
Dyclonine
HNA
ALDH
HNE
GST
HNE-GSH
GSH
Cysteine
Cystine
xCT
INTRACELLULAR
EXTRACELLULAR
CELL MEMBRANE
Cystine
Sulfasalazine

FIG. 12

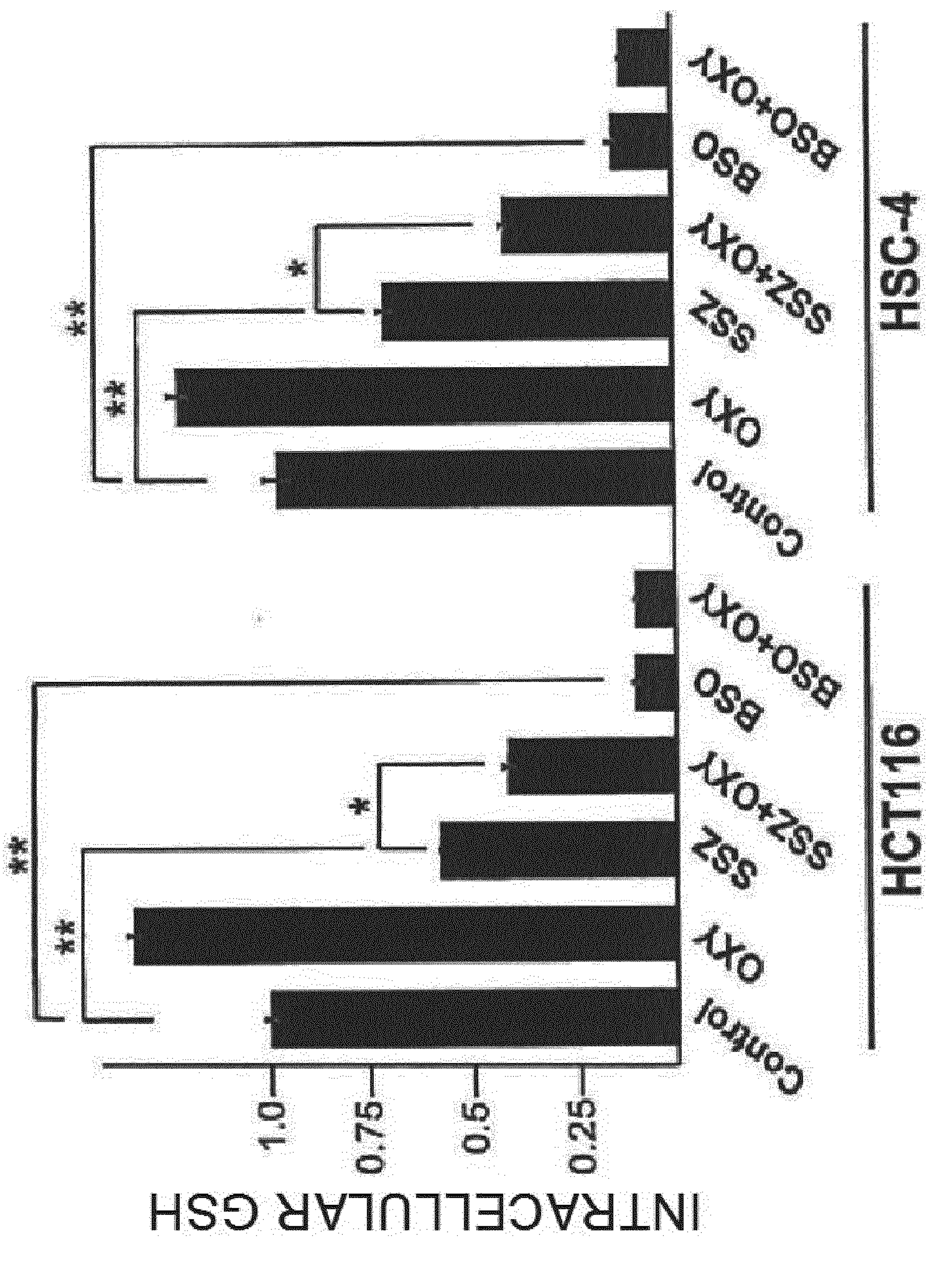
INTRACELLULAR GSH
1.0
0.75
0.5
0.25
Control
OXY
SSZ
SSZ+OXY
BSO
BSO+OXY
HCT116
HSC-4
**
*

FIG. 13

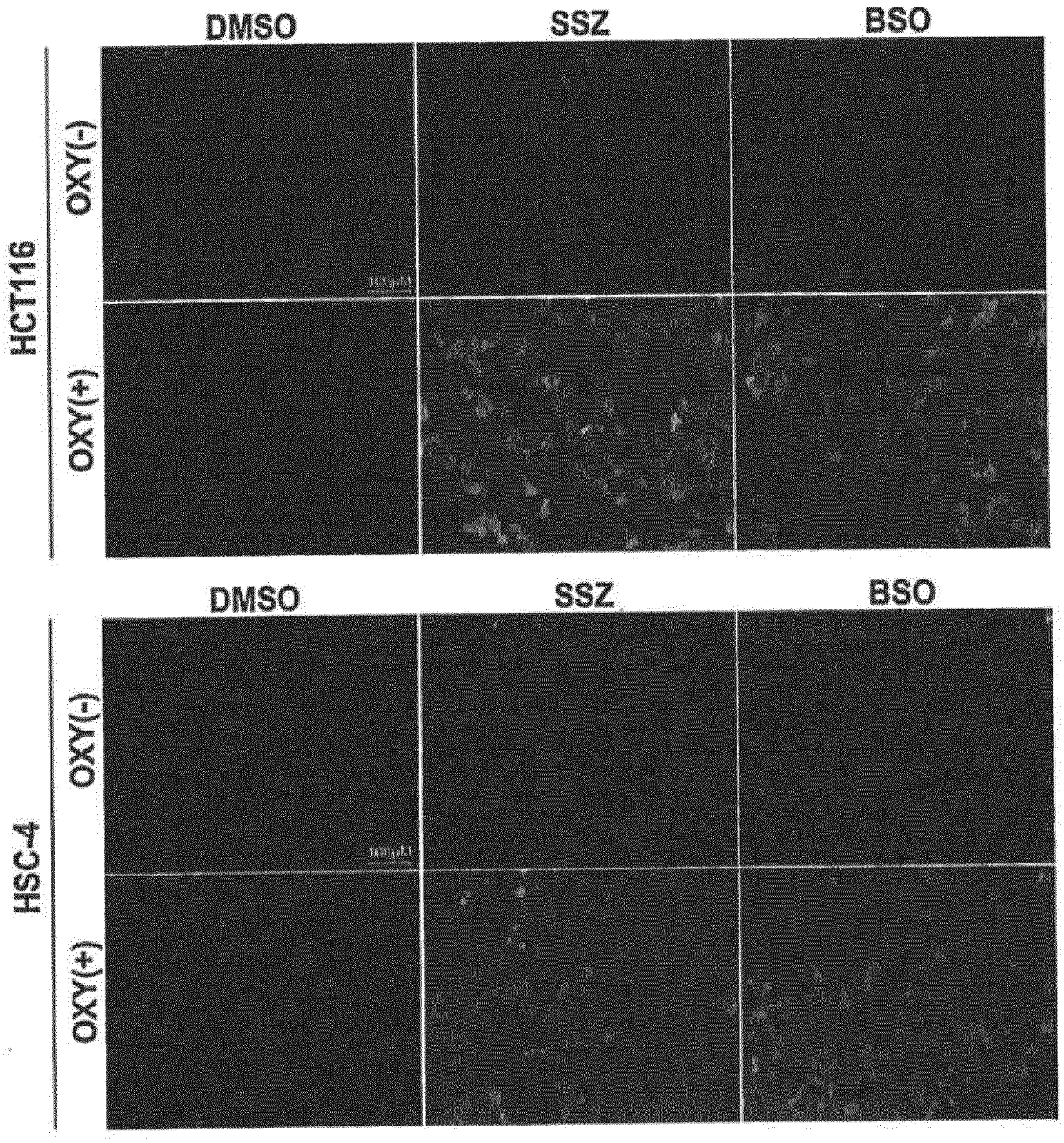


FIG. 14

FIG. 15A

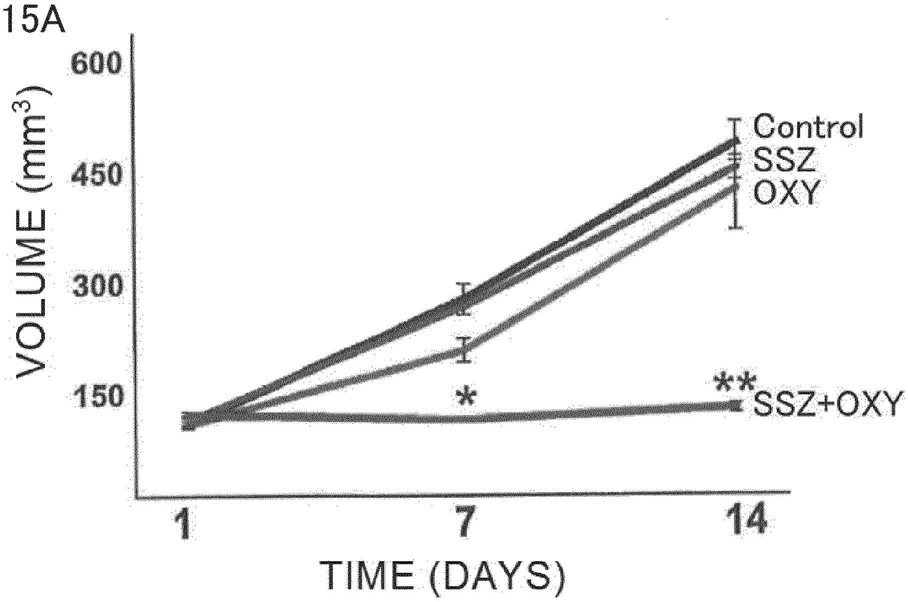


FIG. 15B

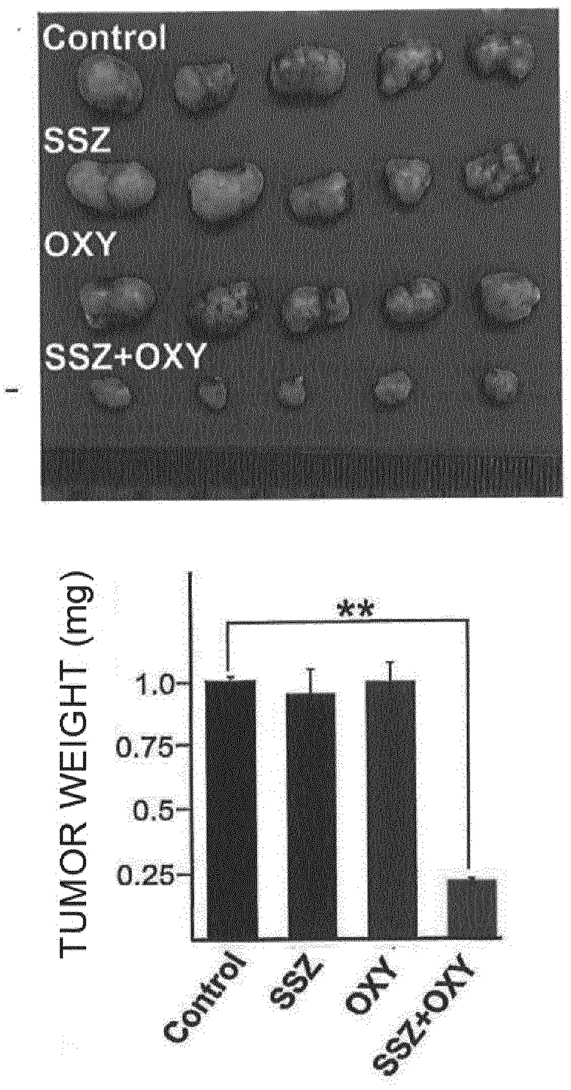

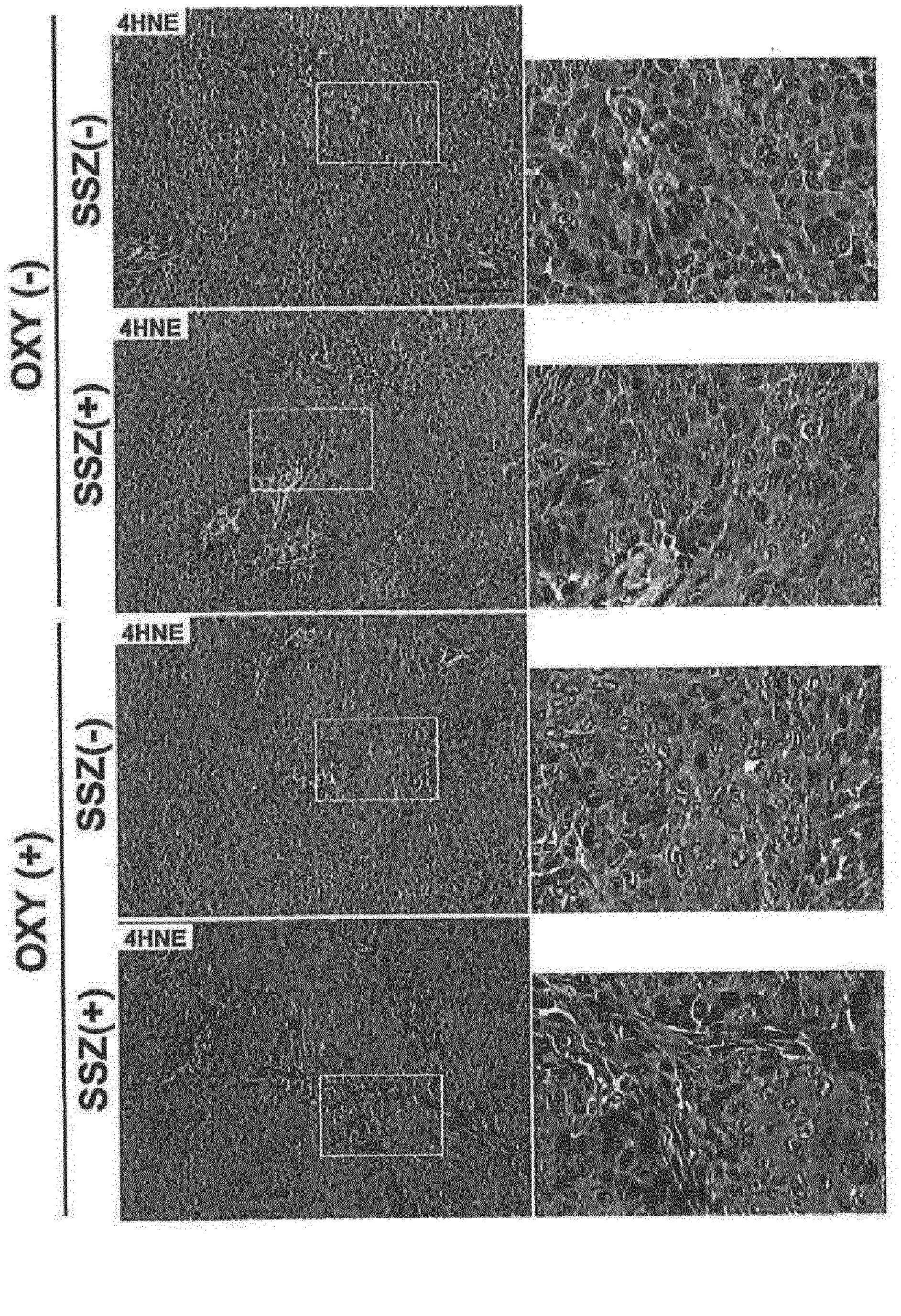


FIG. 16

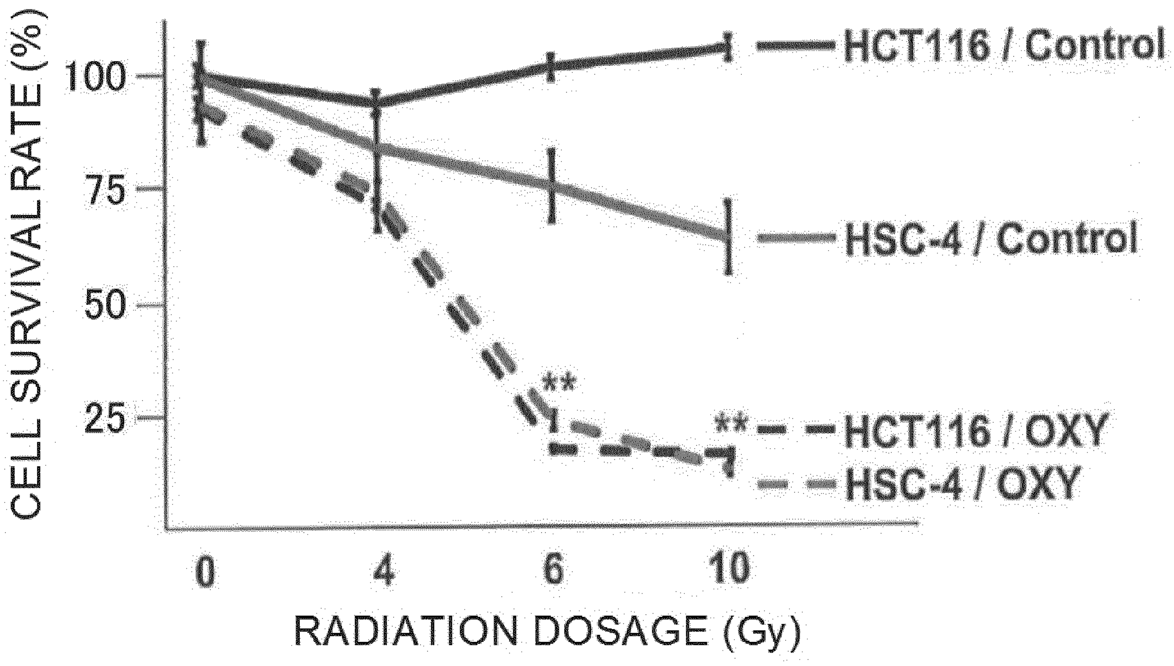
CELL SURVIVAL RATE (%)
100
75
50
25
HCT116 / Control
HSC-4 / Control
**
**
HCT116 / OXY
HSC-4 / OXY
0
4
6
10
RADIATION DOSAGE (Gy)

FIG. 17

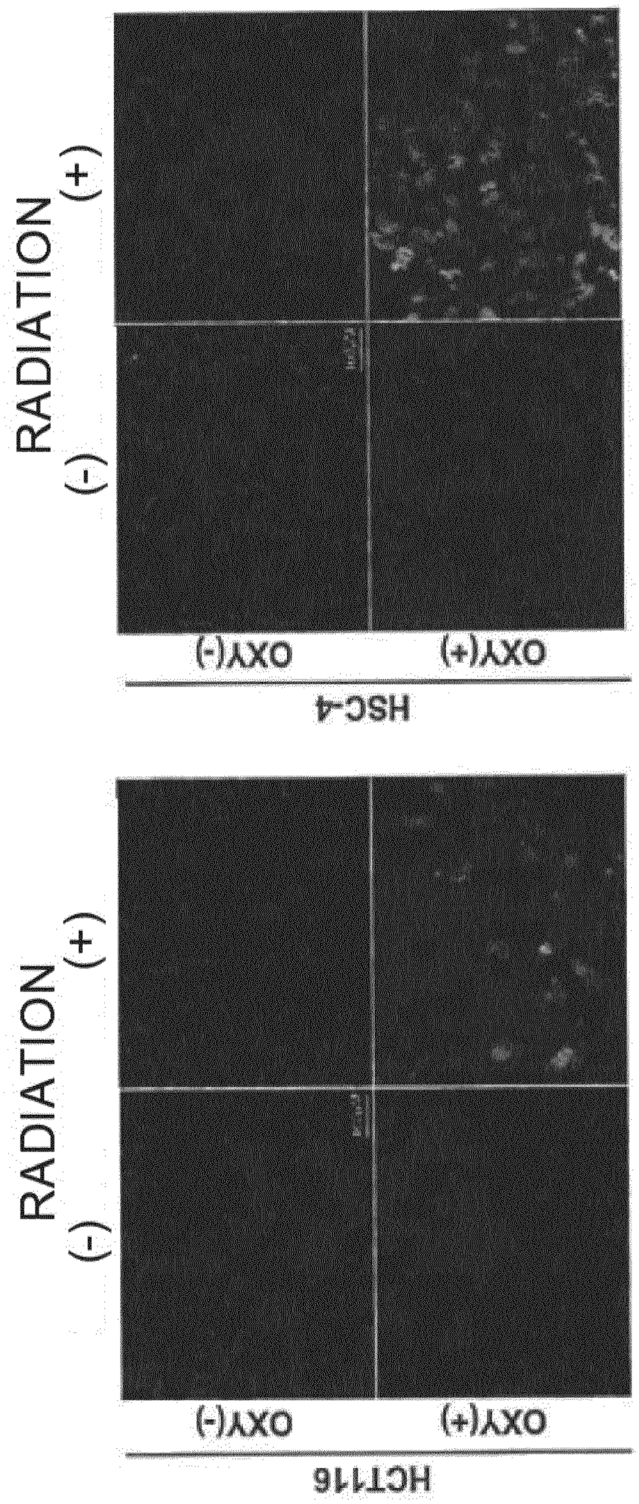


FIG. 18

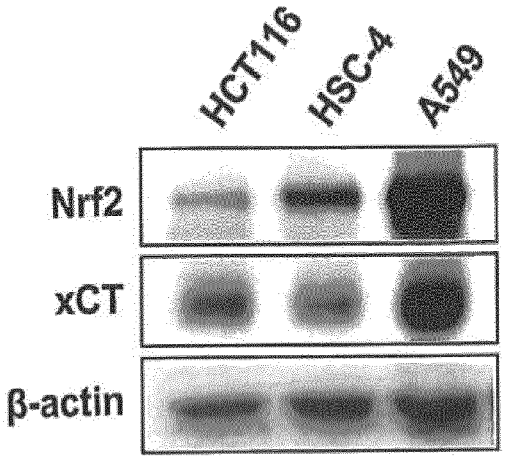


FIG. 19A

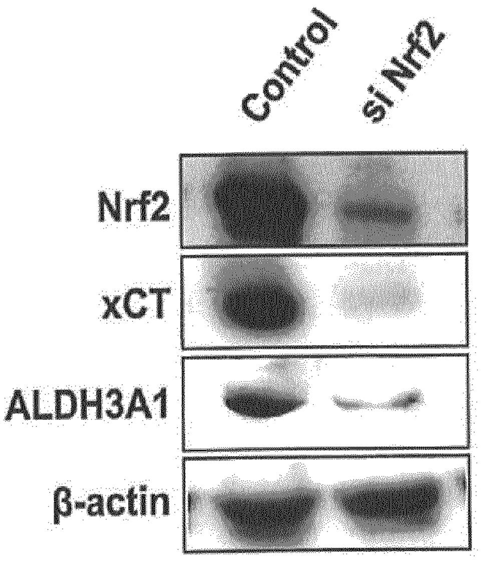


FIG. 19B

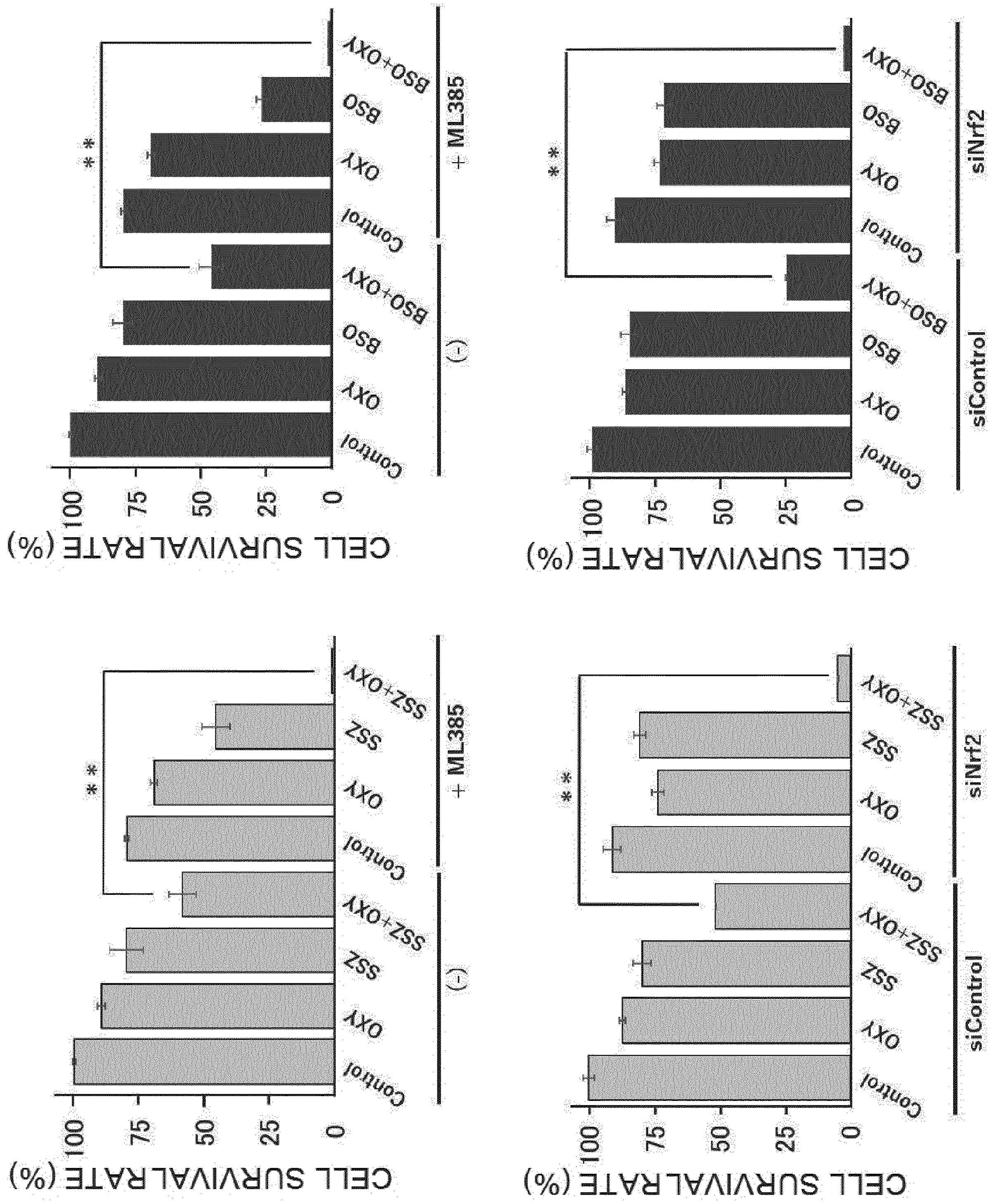
CELL SURVIVAL RATE (%)
0
25
50
75
100
Control
OXY
BSO
BSO+OXY
SSZ
SSZ+OXY
**
(-)
+ ML385
siControl
siNrf2

FIG. 20

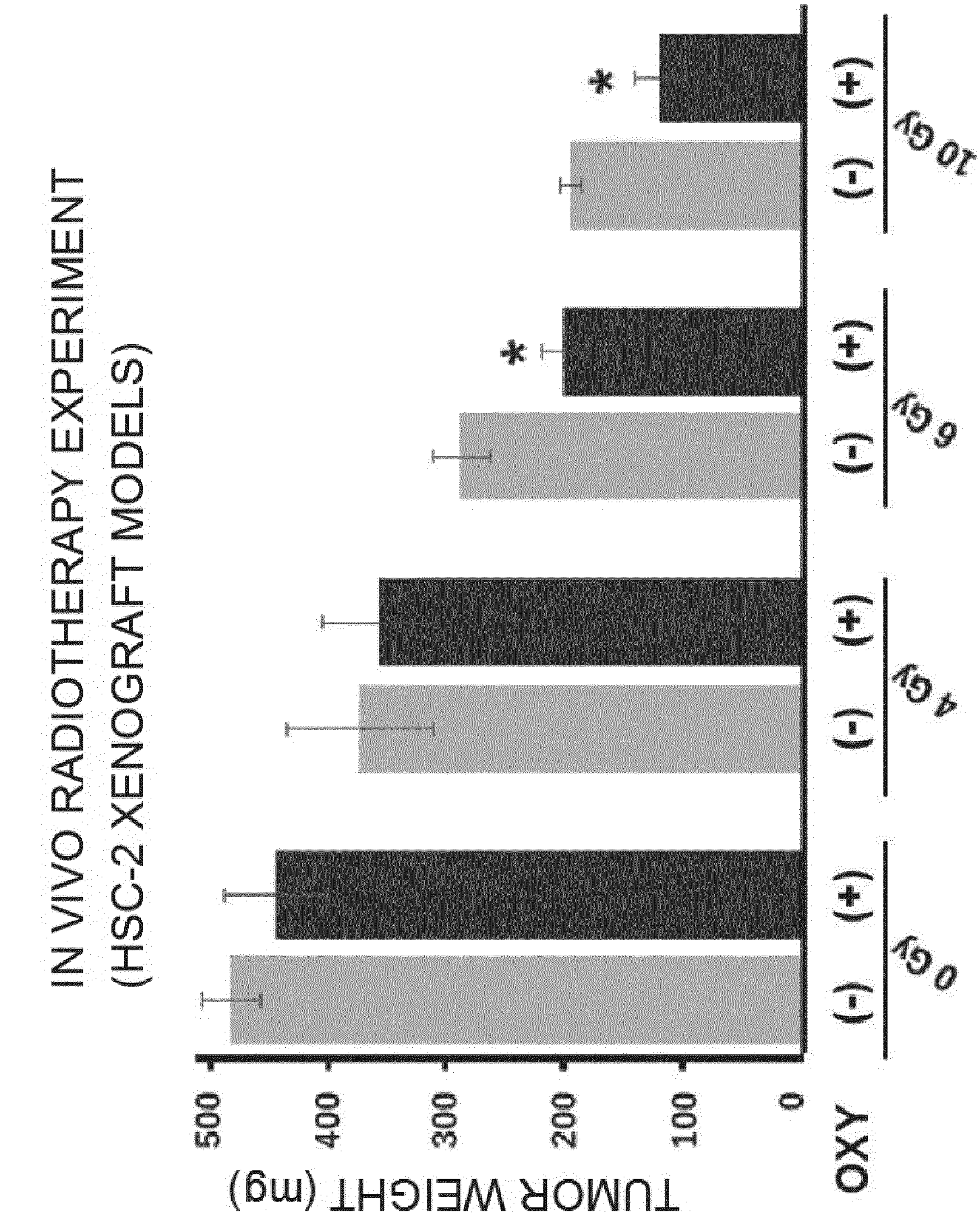
IN VIVO RADIOTHERAPY EXPERIMENT
(HSC-2 XENOGRAFT MODELS)
TUMOR WEIGHT (mg)
500
400
300
200
100
0
OXY
(-)
(+)
0 Gy
(-)
(+)
4 Gy
(-)
(+)
*
6 Gy
(-)
(+)
*
10 Gy

FIG. 21

## INTERNATIONAL SEARCH REPORT

International application No. PCT/JP2020/018572

**A. CLASSIFICATION OF SUBJECT MATTER**

A61K 45/00(2006.01)i; A61K 45/06(2006.01)i; A61P 35/00(2006.01)i; A61P 43/00(2006.01)i; C07K 16/18(2006.01)i; C12N 15/113(2010.01)i; C12Q 1/02(2006.01)i; C12N 9/99(2006.01)i; G01N 33/15(2006.01)i; G01N 33/50(2006.01)i; G01N 33/53(2006.01)i; A61K 31/12(2006.01)i; A61K 31/135(2006.01)i; A61K 31/137(2006.01)i; A61K 31/198(2006.01)i; A61K 31/4453(2006.01)i; A61K 31/635(2006.01)i

FI: A61K31/135 ZNA; C12N9/99; A61P35/00; A61K31/12; A61K31/4453; A61K45/06; A61K31/635 ZNA; A61K31/137; A61P43/00 111; G01N33/53 M; C12N15/113 110Z; C07K16/18; A61K31/198; C12Q1/02; A61P43/00 121; G01N33/50 Z; G01N33/15 Z; A61K45/00

According to International Patent Classification (IPC) or to both national classification and IPC

**B. FIELDS SEARCHED**

Minimum documentation searched (classification system followed by classification symbols)

A61K45/00; A61K45/06; A61P35/00; A61P43/00; C07K16/18; C12N15/113; C12Q1/02; C12N9/99; G01N33/15; G01N33/50; G01N33/53; A61K31/12; A61K31/135; A61K31/137; A61K31/198; A61K31/4453; A61K31/635

Documentation searched other than minimum documentation to the extent that such documents are included in the fields searched

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | OKAZAKI, S. et al., "Synthetic lethality of the ALDH3A1 inhibitor dyclonine and xCT inhibitors in glutathione deficiency-resistant cancer cells", Oncotarget, September 2018, vol. 9, no. 73, pp. 33832-33843 abstract, each drawing | 1-5, 7-10, 12-23<br>1-45 |
| Y | CANTONI, O. et al., "Similarity in the acute cytotoxic response of mammalian cells to mercury (II) and X-rays: DNA damage and glutathione depletion", Biochemical and Biophysical Research Communications, 1982, vol. 108, no. 2, pp. 614-619 abstract | 24-33 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 July 2020 (02.07.2020) | 14 July 2020 (14.07.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2020/018572

C (Continuation). DOCUMENTS CONSIDERED TO BE RELEVANT

| Category* | Citation of document, with indication, where appropriate, of the relevant passages | Relevant to claim No. |
|---|---|---|
| Y | HABIB, E. et al., "Expression of xCT and Activity of System Xe(-) Are Regulated by NRF2 in Human Breast Cancer Cells in Response to Oxidative Stress", Redox Biology, 2015, vol. 5, pp. 33-42 abstract, each drawing | 34-45 |
| Y | KHANNA, M., et al., "Discovery of a Novel Class of Covalent Inhibitor for Aldehyde Dehydrogenases", The Journal of Biological Chemistry, 2011, vol. 286, no. 50, pp. 43486-43494 fig. 4, page 43492, right column, paragraph [0003] | 1-45 |
| P, X<br>P, Y | WO 2019/098288 A1 (KEIO GIJUKU) 23.05.2019 (2019-05-23) claims, examples, each drawing | 1-5, 7-10, 12-23<br>24, 26-39, 41-43, 45 |
| P, X | OTSUKI, Y. et al., "Vasodilator oxyfedrine inhibits aldehyde metabolism and thereby sensitizes cancer cells to xCT-targeted therapy", Cancer Science, 04 January 2020, vol. 111, no. 1, pp. 127-136 examples, each drawing | 1-45 |

Form PCT/ISA/210 (continuation of second sheet) (January 2015)

**INTERNATIONAL SEARCH REPORT**

International application No.
PCT/JP2020/018572

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**

International application No.
PCT/JP2020/018572

<Continuation of Box No. III>

Document 1: OKAZAKI, S. et al., "Synthetic lethality of the ALDH3A1 inhibitor dyclonine and xCT inhibitors in glutathione deficiency-resistant cancer cells", Oncotarget, September 2018, vol. 9, no. 73, pp. 33832-33843 abstract, each drawing

The claims are classified into the following three inventions.

(Invention 1) Claims 1-23

Document 1 discloses an "antitumor agent containing an xCT inhibitor that is administered simultaneously with an aldehyde dehydrogenase inhibitor or dyclonine", a "compounding agent containing an xCT inhibitor and an aldehyde dehydrogenase inhibitor or dyclonine", and a "process for simultaneously administering an aldehyde dehydrogenase inhibitor or dyclonine and an xCT inhibitor to a tumor cell *in vitro*", claims 1-5, 7-10, and 12-23 lack novelty in light of document 1, and thus claims 1-5, 7-10, and 12-23 do not have a special technical feature.

However, claim 6, which is dependent on claim 1, has the special technical feature in which the "compound is oxyfedrine", and claim 11 also has the same technical feature as claim 6.

Therefore, claims 1-23 are classified as invention 1.

(Invention 2) Claims 24-33

Claim 24 cannot be said to share an identical or corresponding technical feature with claim 6 classified as invention 1.

Moreover, claim 24 is not substantially identical or equivalent to any of the claims classified as invention 1.

Therefore, claim 24 cannot be classified as invention 1.

In addition, claims 24-33 have the special technical feature of an "antitumor agent which is used in combination with radiation, contains an aldehyde dehydrogenase inhibitor, and is used for radiation therapy", and are thus classified as invention 2.

(Invention 3) Claims 34-45

Claim 34 cannot be said to share an identical or corresponding technical feature with claim 6 classified as invention 1 or claim 24 classified as invention 2.

Moreover, claim 34 is not substantially identical or equivalent to any of the claims classified as invention 1 or invention 2.

Therefore, claim 34 cannot be classified as any of invention 1 and invention 2.

In addition, claims 34-45 have the special technical features of "containing an inhibitor of an xCT expression-enhancing action of Nrf2", "containing a reagent for detecting the expression of an Nrf2 gene", and "containing a reagent for detecting a mutation in a Keap1 gene or an Nrf2 gene", and are thus classified as invention 3.

Form PCT/ISA/210 (extra sheet) (January 2015)

**INTERNATIONAL SEARCH REPORT**
Information on patent family members

International application No.
PCT/JP2020/018572

| Patent Documents referred in the Report | Publication Date | Patent Family | Publication Date |
|---|---|---|---|
| WO 2019/098288 A1 | 23 May 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2012144498 A
- JP 2019091358 A
- JP 2019200094 A
- WO 9508563 A
- WO 9640205 A
- WO 9954346 A

### Non-patent literature cited in the description

- *Cancer Cell,* 08 March 2011, vol. 19 (3), 387-400
- *Leukemia,* 2001, vol. 15, 1633-1640

SEQUENCE LISTING

<110> Keio University

<120> Anti-tumor agent and combination agent

<130> KO190594

<160> 10

<170> PatentIn version 3.5

<210> 1
<211> 21
<212> DNA
<213> Artificial Sequence

<220>
<223> siRNA

<400> 1
uucuccgaac gugucacgut t 21

<210> 2
<211> 21
<212> DNA
<213> Artificial Sequence

<220>
<223> siRNA

<400> 2
acgugacacg uucggagaat t 21

<210> 3
<211> 21
<212> DNA
<213> Artificial Sequence

<220>
<223> siRNA

<400> 3
agaaaucugg aggucauuat t 21

<210> 4
<211> 21
<212> DNA
<213> Artificial Sequence

<220>
<223> siRNA

<400> 4
uaaugaccuc cagauuucut t 21

<210> 5
<211> 21
<212> DNA

<213> Artificial Sequence

<220>
<223> siRNA

<400> 5
ccagaacauu acaaauaaut t 21

<210> 6
<211> 21
<212> DNA
<213> Artificial Sequence

<220>
<223> siRNA

<400> 6
auuauuugua auguucuggt t 21

<210> 7
<211> 21
<212> DNA
<213> Artificial Sequence

<220>
<223> siRNA

<400> 7
uucuccgaac gugucacgut t 21

<210> 8
<211> 21
<212> DNA
<213> Artificial Sequence

<220>
<223> siRNA

<400> 8
acgugacacg uucggagaat t 21

<210> 9
<211> 21
<212> DNA
<213> Artificial Sequence

<220>
<223> siRNA

<400> 9
uccuacugug augugaaaut t 21

<210> 10
<211> 21
<212> DNA
<213> Artificial Sequence

<220>

<223> siRNA

<400> 10
auuucacauc acaguaggag c 21